# EUROPEAN PATENT APPLICATION

(11) **EP 2 210 887 A1**
(43) Date of publication of application: **28.07.2010**
(21) Application number: 09150557.8
(22) Date of filing: 14.01.2009
(51) Int. Cl.: C07D 403/04, A61K 8/49, A61K 31/53, A61Q 17/04

(54) **Bis resorcinyl triazine derivatives as protecting agents against UV radiation**

(71) Applicant: ISDIN, S.A., 08006 Barcelona (ES)
(72) Inventor: Trullàs Cabanas, Carles, 08006 Barcelona (ES); Corbera Arjona, Jordi, 08041 Barcelona (ES); Gorchs Capa, Olga, 08006 Barcelona (ES); Jiménez Alonso, Óscar, 08006 Barcelona (ES)
(74) Representative: ZBM Patents

(57) **Abstract**

The present invention relates to new compounds of general formula (I) where R₁ is a ring system containing C and N, being at least one of them different from C; optionally being substituted; R₂ and R₂' are radicals, same or different, independently selected from linear or branched (C₁-C₄)alkyl, and-O-(C₁-C₄)alkyl; R₃ and R₃' are radicals, same or different, independently selected from hydrogen, (C₁-C₁₈)acyl radical, linear or branched (C₁-C₁₈)alkyl, and linear or branched (C₂-C₁₈)alkenyl; wherein the radical is optionally substituted. The physicochemical properties of said compounds allow them to be used as protecting agents against UV radiation.

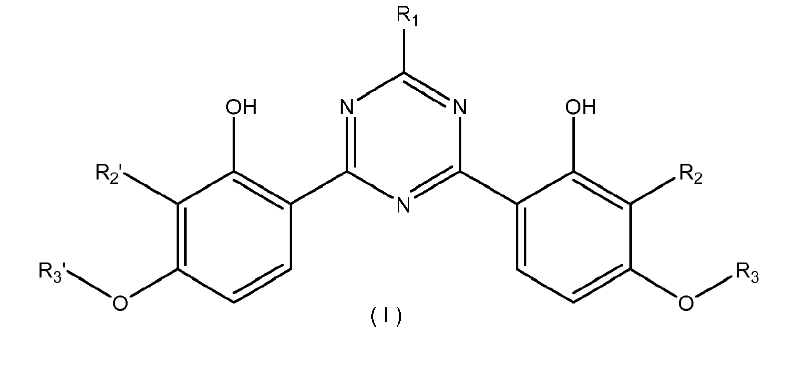

## Description

### FIELD OF THE INVENTION

The present invention is related to the cosmetic, dermatological and pharmaceutical fields. In particular, the present invention relates to new bis-resorcinyl triazine derivatives which are useful as protecting agents against UV radiation, together with their use for manufacturing cosmetic, dermatological and pharmaceutical formulations against UV radiation.

### BACKGROUND ART

The electromagnetic spectrum of ultraviolet light can be subdivided in a number of ways. The ISO standard on determining solar irradiances (ISO-21348) describes the following ranges:

| **Spectral category** | **Spectral sub-category** | **Wavelength range (nm)** |
|---|---|---|
| Ultraviolet | UV-C | 100 ≤ λ ≤ 280 |
| | UV-B | 280 ≤ λ ≤ 315 |
| | UV-A | 315 ≤ λ ≤ 400 |

It is known that light radiation with wavelengths of from 280 nm to 400 nm promotes tanning of the human epidermis and that irradiation with wavelengths of from 280 to 320 nm, i.e. UV-B radiation, causes erythemas and skin burns which may be harmful to the development of natural tanning, this UV-B radiation must therefore be screened from the skin.

It is also known to this art that UV-A radiation, with wavelengths of from 320 nm to 400 nm, which causes tanning of the skin, can also adversely affect it, in particular in the case of sensitive skin or of skin continually exposed to solar radiation. UV-A rays cause a loss in the elasticity of the skin and the appearance of wrinkles, resulting in premature aging. Such irradiation promotes the triggering of the erythemal reaction of accentuates this reaction in certain individuals and can even be the cause of phototoxic or photoallergic reactions. It is therefore desirable to also screen out UV-A radiation.

Against the damage that UV-A and UV-B radiation can cause, people have various natural protective systems in their skins that either absorb or deflect the radiation, such as melanin, hair, the fatty layer of the skin, etc.

In this respect, solar filters are currently used in order to reduce the effects of solar radiation. Such solar filters are compounds that are applied on the skin, lips, nails or hair and can be found in cosmetic, dermatological and pharmaceutical formulations as well as in other cosmetic products for protecting against solar radiation, preventing the decomposition of active ingredients or radiation-sensitive components.

Research has been carried out in recent years into compounds whose physicochemical properties make them more effective as solar filters. Accordingly, a wide variety of cosmetic, dermatological and/or pharmaceutical compositions for the photoprotection (UV-A and/or UV-B) of human skin are known to the art.

Different bis-resorcinyl triazine derivatives are known in the art, thus in WO 03/075875 are disclosed compositions that absorb UV radiation including a hydroxyphenyltriazine compound are disclosed.

WO 06/064366 describes 6-(1H-pyrazol-5-yl)-1,3,5-triazine derivatives as protective agents against UV radiation.

EP1891050 describes 1-benzylpyrrol triazine derivatives as protective agents against UV radiation.

Despite the great effort in obtaining wide efficient solar filters, there is a need of finding new compounds whose physicochemical properties make them suitable as solar filters for offering simultaneous protection against UV-A and UV-B radiation.

### SUMMARY OF THE INVENTION

The inventors of the present invention have found that the compounds of general formula (I) absorb in the ultraviolet radiation range of both types A and B, with said derivatives therefore being useful as UV radiation absorbing agents and effective simultaneously in protecting against UV-A and UV-B radiation.

It has been found that the presence of the radicals R₂ and R₂' in the resorcinyl moiety results in unexpected changes in the UV profile, in which the adequate combination of R₁, R₂, R₂', R₃, and R₃' results in a high solubility profile, and an excellent photo-stability of the compound alone and in combination with other UV filters. Furthermore, the synthetic approach showed to be satisfactory, which could in turn, made scalable and more easily the process to industrialize the synthesis of these compounds.

A first aspect of the invention relates to compounds of general formula (I), an stereoisomer thereof, a pharmaceutically, cosmetically or dermatologically acceptable salt thereof or a pharmaceutically, cosmetically or dermatologically acceptable solvate thereof: 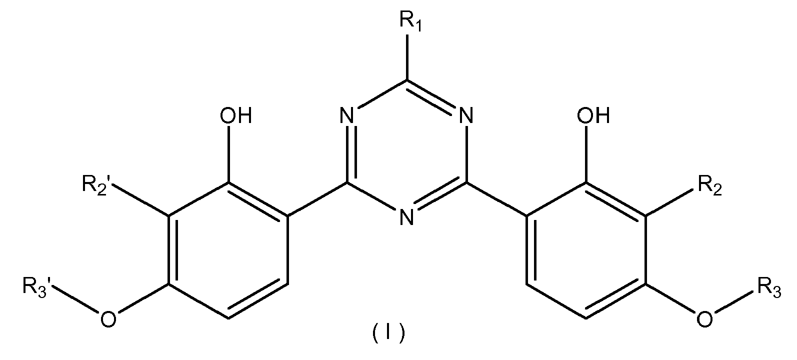 wherein:
R₁ is a ring system of formula (II): 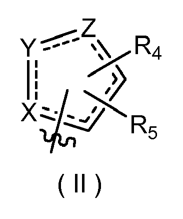
wherein:
X, Y and Z are each one independently selected from C and N, being at least one of them different from C;
the dotted line represents the presence or absence of a double bond;
the wavy line shows the attach point of the moiety;
R₄ and R₅ are independently selected from H, halogen, nitro, cyano, linear or branched (C₁-C₁₈)alkyl, linear or branched (C₂-C₁₈)alkenyl, -OR₆, -COR₆, -COOR₆, -OC(O)R₆, -C(O)NR₇R₈, -R₉NR₇R₈, -R₉PO(OR₁₀)₂ -S-R₁₁, -SO-R₁₁, -SO₂-R₁₁, -SO₃M, -NHSO₂-R₁₁, -SO₂-NR₇R₈, -NR₇R₈, and an optionally substituted aromatic or heteroaromatic ring having 5-6 members; being the substituent selected from the group consisting of halogen, nitro, cyano, linear or branched (C₁-C₈)alkyl, linear or branched (C₂-C₈)alkenyl, -OR₆, -COR₆, -COOR₆, -OC(O)R₆, -C(O)NR₇R₈, -R₉NR₇R₈, -R₉PO(OR₁₀)₂, -S-R₁₁, -SO-R₁₁, -SO₂-R₁₁, -SO₃M, -NHSO₂R₁₁, -SO₂-NR₇R₈, and -NR₇R₈;
or R₄ and R₅ taken together with the atoms to which they are attached form one of the known ring of 6 members, saturated, partially unsaturated or aromatic, wherein each member is independently selected from C, and N; thereby the ring system of formula (II) is a bicyclic system;
the bicyclic system of formula (II) being optionally substituted by at least one radical selected from the group consisting of halogen, nitro, cyano, linear or branched (C₁-C₁₈)alkyl, linear or branched (C₂-C₁₈)alkenyl, -OR₆, -COR₆, -COOR₆, -OC(O)R₆, -C(O)NR₇R₈, -R₉NR₇R₈, -R₉PO(OR₁₀)₂, -S-R₁₁, -SO-R₁₁, -SO₂-R₁₁, -SO₃M, -NHSO₂R₁₁, -SO₂-NR₇R₈, -NR₇R₈, and an optionally substituted aromatic or heteroaromatic ring having 5-6 members; being the substituent selected from the group consisting of halogen, nitro, cyano, linear or branched (C₁-C₈)alkyl, linear or branched (C₂-C₈)alkenyl, -OR₆, -COR₆, -COOR₆, -OC(O)R₆, -C(O)NR₇R₈, -R₉NR₇R₈, -R₉PO(OR₁₀)₂ -S-R₁₁, -SO-R₁₁, -SO₂-R₁₁, -SO₃M, -NHSO₂R₁₁, -SO₂-NR₇R₈, and -NR₇R₈;
R₆, R₇, R₈, R₁₀ and R₁₁ are radicals, same or different, independently selected from the group consisting of hydrogen, and linear or branched (C₁-C₆)alkyl;
R₉ is a biradical selected from the group consisting of linear or branched (C₁-C₆)alkyl;
R₂ and R₂' are radicals, same or different, independently selected from the group consisting of linear or branched (C₁-C₄)alkyl, and -O-(C₁-C₄)alkyl ;
R₃ and R₃' are radicals, same or different, independently selected from the group consisting of hydrogen, (C₁-C₁₈)acyl radical, linear or branched (C₁-C₁₈)alkyl, and linear or branched (C₂-C₁₈)alkenyl; wherein the radical is optionally substituted with at least one radical selected from the group consisting of OH, -SO₃M, -N(R₁₂)₃⁺ X⁻ group and a group of general formula (III) 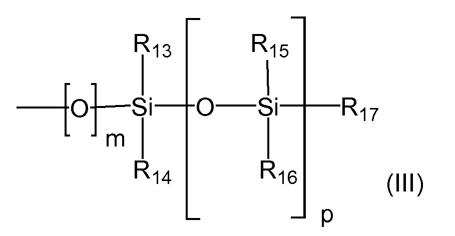
in which
m= 0 or 1;
p= 0, 1, 2, 3 or 4;
R₁₃, R₁₄, R₁₅, R₁₆ and R₁₇ are independently selected from the group consisting of (C₁-C₆)alkyl radical; an (C₁-C₆)alkoxy radical and an -OSi(R₁₈)₃ radical;
R₁₈ represents an (C₁-C₆)alkyl radical or an (C₁-C₆)alkoxy radical;
R₁₂ represents an (C₁-C₆)alkyl radical; and
M is H, Na, K or triethanolamine;
X⁻ represents an anion of a dermatologically, cosmetically, and/or pharmaceutically acceptable acid.

Another aspect of the present invention is the method to prepare a compound according to the first aspect of the invention.

Also part of the present invention, therefore, are cosmetic, dermatological or pharmaceutical formulations that include one or more compounds of general formula (I), according to the first aspect of the invention, and at least one cosmetically, dermatologically or pharmaceutically acceptable carrier or excipients. Preferably, the formulations further include at least one organic, micronized organic or inorganic filter against solar radiation, or one active substance.

A fourth aspect of the present invention relates to the use of the compounds according to the first aspect of the invention
a) in a cosmetic, dermatological or pharmaceutical formulation as a UV radiation filtering agent;
b) for manufacturing a formulation for protection of the skin, lips and/or related tissues of a mammal against solar radiation;
c) for manufacturing a formulation for preventive use, as a coadjuvant in the treatment of pathologies caused by ultraviolet radiation on the skin, lips and/or related tissues of a mammal, such as polymorphous light eruptions, photoageing, actinic keratosis, vitiligo, solar urticaria, chronic actinic dermatitis and xeroderma pigmentosum.

These above mentioned aspect of the invention can also be formulated in an equivalent wording as at least one compound or mixture of compounds according to the first aspect of the invention, for use in a cosmetic, dermatological, pharmaceutical or veterinary formulation as a UV radiation filtering agent, protector of the skin, lips and/or related tissues of a mammal against solar radiation, as well as for preventive use, as a coadjuvant in the treatment of pathologies caused by ultraviolet radiation on the skin, lips and/or related tissues of a mammal, such as polymorphous light eruptions, photoageing, actinic keratosis, vitiligo, urticaria solar, chronic actinic dermatitis and xeroderma pigmentosum.

Preferably, said formulation is applied topically.

In a preferred embodiment said mammal is a human being.

The properties of the compounds of general formula (I) make these compounds also useful as photostabilisers of synthetic polymers and as solar filter for textile fibres. Therefore, another aspect of the present invention relates to the use of the compounds according to the first aspect of the invention as photostabilisers of synthetic polymers and as solar filter for textile fibres.

### DETAILED DESCRIPTION OF THE INVENTION:

According to an embodiment of the present invention, the compounds of formula (I) are those wherein R₁ is selected from the group consisting of 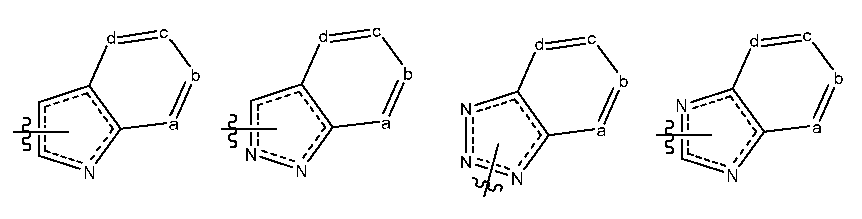 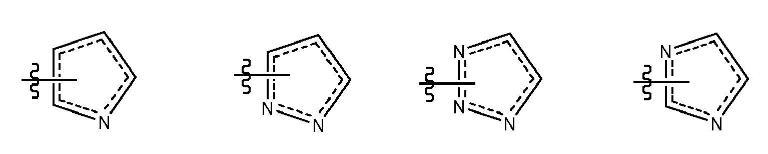 wherein a, b, c, and d are independently selected from C, and N;
the wavy line shows the attach point of the moiety;
wherein R₁ is optionally substituted by at least one radical selected from the group consisting of halogen, nitro, cyano, linear or branched (C₁-C₈)alkyl, linear or branched (C₂-C₈)alkenyl, -OR₆, -COR₆, -COOR₆, OC(O)R₆, -C(O)NR₇R₈, -R₉NR₇R₈, -R₉PO(OR₁₀)₂ -S-R₁₁, -SO-R₁₁, -SO₂-R₁₁, -SO₃M, -NHSO₂R₁₁, -SO₂-NR₇R₈, -NR₇R₈, and an optionally substituted aromatic or heteroaromatic ring having 5-6 members; being the substituent selected from the group consisting of halogen, nitro, cyano, linear or branched (C₁-C₈)alkyl, linear or branched (C₂-C₈)alkenyl, -OR₆, -COR₆, -COOR₆, -OC(O)R₆, -C(O)NR₇R₈, -R₉NR₇R₈, -R₉PO(OR₁₀)₂, -S-R₁₁, -SO-R₁₁, -SO₂-R₁₁, -SO₃M, -NHSO₂-R₁₁, -SO₂-NR₇R₈, and -NR₇R₈;
being R₆-R₁₁ and M as defined above.

According to a preferred embodiment, R₁ is selected from the group consisting of

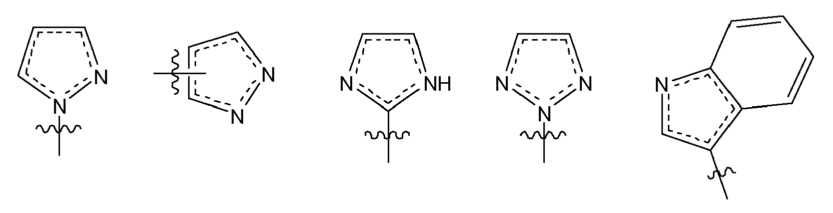

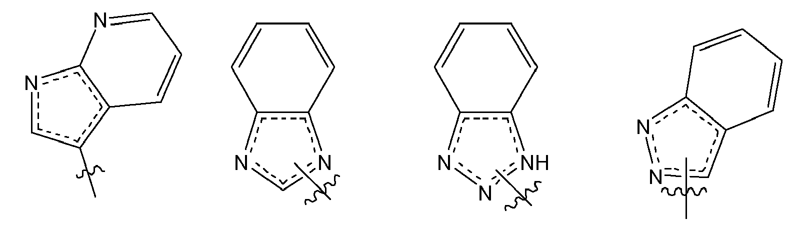

In another preferred embodiment, this invention relates to a compound of formula (I) wherein R₁ is selected from the group consisting of

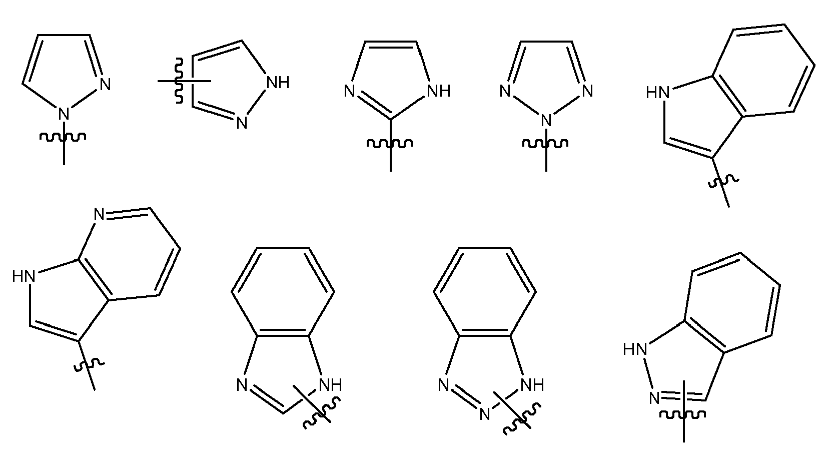

In more preferred embodiment, this invention relates to a compound of formula (I) wherein R₁ is substituted by at least one radical selected from the group consisting of halogen, linear or branched (C₁-C₈)alkyl, -OR₆, -NR₇R₈, and an optionally substituted aromatic or heteroaromatic ring having 5-6 members; being the substituent selected from the group consisting of halogen, cyano, linear or branched (C₁-C₈)alkyl, -OR₆, -COR₆, -NR₇R₈, and -SO₃M.

In another more preferred embodiment, R₁ is substituted by at least one radical selected from the group consisting of methyl, ethyl, propyl, *iso*-propyl, OH, methoxy, ethoxy, 2-ethylhexyl, 2-ethylhexyloxy, phenyl optionally substituted by at least one radical selected from OH, methyl, ethyl, methoxy, ethoxy, dimethylamino, -SO₃M, and -NH₂.

According to another embodiment, this invention relates to a compound of formula (I) wherein R₂ and R₂' are radicals, same or different, independently selected from the group consisting of methyl, ethyl, propyl, *iso*-propyl, methoxy, and ethoxy. Particularly preferred, are those wherein R₂ and R₂' are methyl.

According to another embodiment, this invention relates to a compound of formula (I) wherein R₃ and R₃' are radicals, same or different, independently selected from the group consisting of methyl, ethyl, propyl, *iso*-propyl, butyl, *tert*-butyl, *iso*-amyl, hexyl, 2-ethylhexyl, 2-ethyloctyl, 2-butyloctyl, 2-hexyldecyl, -CO-methyl, -CO-ethyl, -CO-propyl, -CO-*iso*-propyl, -CO-butyl, - CO-*tert*-butyl, -CO-hexyl, -CO-octyl; wherein the radical is optionally substituted with at least one radical selected from the group consisting of OH, -SO₃M and -N(Me)₃⁺ X⁻ group or else a group of general formula (III) 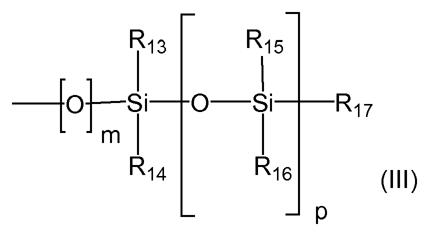 with R₁₃-R₁₇, m and p being as defined above.

In another preferred embodiment, this invention relates to a compound of formula (I) wherein R₃ and R₃' are radicals, same or different, independently selected from the group consisting of methyl, ethyl, propyl, *iso*-propyl, butyl, *tert*-butyl, *iso*-amyl, hexyl, 2-ethylhexyl, 2-butyloctyl, 2-hexyldecyl.

Particularly preferred are:
(1) 2-(1-methyl-pyrazol-5-yl)-4,6-bis[4-(2-ethylhexyloxy)-3-methyl-2-hydroxy-phenyl]-1,3,5-triazine.
(2) 2-(1-methyl-pyrazol-5-yl)-4,6-bis[4-(2-butyloctyloxy)-3-methyl-2-hydroxy-phenyl]-1,3,5-triazine.
(3) 6,6'-(6-(1-methyl-1H-indol-3-yl)-1,3,5-triazine-2,4-diyl)bis(3-(2-ethylhexyloxy)-2-methylphenol).
(4) 6,6'-(6-(1-methyl-1H-indol-3-yl)-1,3,5-triazine-2,4-diyl)bis(3-(2-butyloctyloxy)-2-methylphenol).
(5) 6,6'-(6-(1-(2-ethylhexyl)-1H-indol-3-yl)-1,3,5-triazine-2,4-diyl)bis(3-(2-ethylhexyloxy)-2-methylphenol).
(6) 6,6'-(6-(1-(2-ethylhexyl)-1H-indol-3-yl)-1,3,5-triazine-2,4-diyl)bis(3-(2-butyloctyloxy)-2-methylphenol).
(7) 6,6'-(6-(1-(2-ethylhexyl)-5-methoxy-1H-indol-3-yl)-1,3,5-triazine-2,4-diyl)bis(3-(2-ethylhexyloxy)-2-methylphenol).
(8) 6,6'-(6-(1-(2-ethylhexyl)-2-methyl-1H-indol-3-yl)-1,3,5-triazine-2,4-diyl)bis(3-(2-ethylhexyloxy)-2-methylphenol).
(9) 6,6'-(6-(1-(2-ethylhexyl)-5-methoxy-2-methyl-1H-indol-3-yl)-1,3,5-triazine-2,4-diyl)bis(3-(2-ethylhexyloxy)-2-methylphenol).
(10) 6,6'-(6-(1-(2-ethylhexyl)-5-(4-methoxyphenyl)-1H-indol-3-yl)-1,3,5-triazine-2,4-diyl)bis(3-(2-ethylhexyloxy)-2-methylphenol).
(11) 6,6'-(6-(1-(2-ethylhexyl)-5-phenyl-1H-indol-3-yl)-1,3,5-triazine-2,4-diyl)bis(3-(2-ethylhexyloxy)-2-methylphenol).
(12) 6,6'-(6-(1H-pyrazol-1-yl)-1,3,5-triazine-2,4-diyl)bis(3-(2-ethylhexyloxy)-2-methylphenol).
(13) 6,6'-(6-(4-methyl-3-phenyl-1H-pyrazol-1-yl)-1,3,5-triazine-2,4-diyl)bis(3-(2-ethylhexyloxy)-2-methylphenol).
(14) 6,6'-(6-(4-(4-methoxyphenyl)-1H-pyrazol-1-yl)-1,3,5-triazine-2,4-diyl)bis(3-(2-ethylhexyloxy)-2-methylphenol).

The process described in the Reaction Scheme 1 shows how to obtain the compounds of general formula (I).

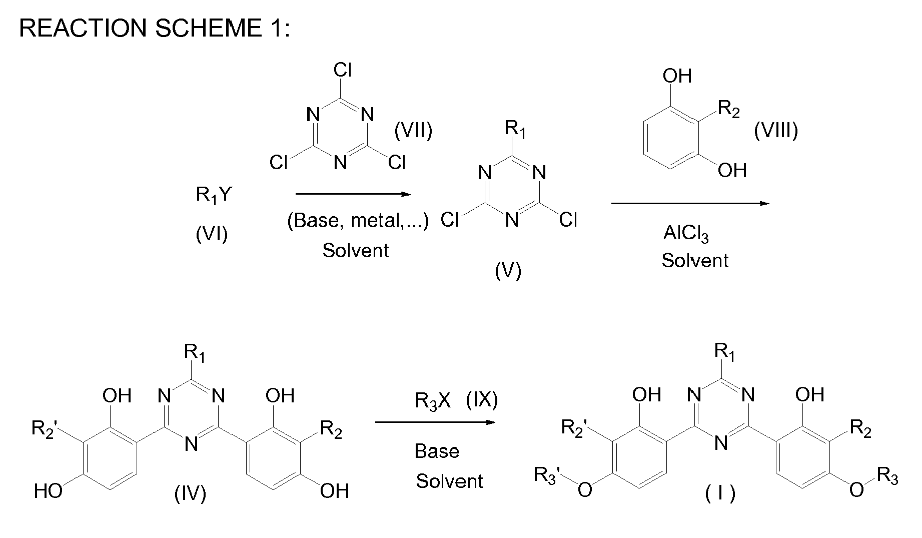

The compound of general formula (V) is obtained by reaction of trichlorotriazine (VII) with the suitable compound R₁Y (VI), where R₁ is as defined above and Y is hydrogen, chloro, bromo, tosyl or mesyl. It would be necessary, depending on the R₁Y nature and/or the way of attachment to the triazine core, the use of a base, such as BuLi, Na₂CO₃, NaH..., or Mg to have the corresponding Grignard reagent, or either a boronic acid or boronic acid derivative, in an inert solvent such as toluene, xylene (mixture of isomers), 1,4-dioxane, diethyl ether, tetrahydrofurane, 1,2-dichloroethane, N-methylpyrrolidone, N,N-dimethylformamide, or sulfolane at a temperature ranging from -78°C to its boiling point.

The compound of general formula (IV) is obtained by Friedel-Crafts reaction of the desired resorcinol moiety (VIII) with the compound of general formula (V) in the presence of a Lewis acid, in particular aluminium chloride, in an inert solvent such as xylene (mixture of isomers), 1,4-dioxane, tetrahydrofurane, N-methylpyrrolidone, N,N-dimethylformamide, 1,2-dichloroethane, sulfolane... at a temperature ranging from room temperature to its boiling point.

The etherification process of the *p*-hydroxyl groups that leads the compounds of general formula (I) is carried out by alkylation of the compounds of general formula (IV) with a compound of general formula (IX), where R₃ is as defined above, and X is a leaving group such as chloro, bromo, tosyl or mesyl, in the presence of a base, such as sodium hydroxide, sodium hydride, caesium carbonate, potassium carbonate, sodium carbonate, lithium carbonate, sodium *tert*-butoxide and potassium *tert*-butoxide, in an appropriate polar solvent such as 2-methoxyethanol, 2-ethoxyethanol, 1,4-dioxane, tetrahydrofurane, N-methylpyrrolidone, N,N-dimethylformamide, sulfolane or ethanol, at a temperature that ranges between room temperature and its boiling point.

It is to be understood that the end-product and all the intermediates may but do not have to be purified.

The purification and isolation of the inventive compounds of general formula I may, if required, be carried out by conventional methods known to those skilled in the art, e.g. chromatographic methods or recrystallization.

As it is illustrated in Example 15 the compounds of general formula (I) according to the first aspect of the present invention have physico-chemical properties such as absorption of ultraviolet light, which permits their use as protecting agents against UV radiation. Particularly, the compounds of formula (I) absorb in the ultraviolet radiation range of both types A and B, with said derivatives therefore being useful as UV radiation absorbing agents and effective simultaneously in protecting against UV-A and UV-B radiation.

Also object of the present invention, therefore, are cosmetic, dermatological or pharmaceutical formulations that include one or more compounds of general formula (I), according to the first aspect of the invention, and at least one cosmetically, dermatologically or pharmaceutically acceptable carrier or excipient.

In a preferred embodiment, said cosmetic, dermatological or pharmaceutical formulation also includes at least one organic, micronised organic or inorganic filter against solar radiation. In another preferred embodiment, said formulation also includes at least one active substance.

Said cosmetic, dermatological or pharmaceutical formulation can be adapted to apply to the skin and lips in the form of: a non-ionic vesicular dispersion, emulsion, cream, lotion, gel, aerosol, cream-gel, gel-cream, suspension, dispersion, powder, solid stick, foam, spray, oil, ointment or fluid, among others.

Similarly, said formulation can be adapted to apply to the hair in the form of a shampoo, lotion, gel, fluid, lacquer, foam, dye, emulsion, cream or spray, among others, and on the nails in the form of a nail varnish, oil or gel, among others.

Moreover, the organic, micronised organic and inorganic filters are selected from those acceptable under the country's legislation.

For example, the organic filters can be selected from those approved by the Council of the European Community (revised text of European Directive 76/768/EEC Annex-7. pages 76-81, published on 15.10.2003) and by the U.S. Food and Drug Administration (see, for example, "Food and Drugs, Sunscreen drug products for over-the-counter human use", title 21, volume 5 of the Code of Federal Regulations, revised on 1 April 2004), such as antranilates; camphor derivatives; dibenzoylmethane derivatives; benzotriazole derivatives; diphenylacrylate derivatives; cinnamic derivatives; salicylic derivatives; triazine derivatives such as those described in patents EP-863145, EP-517104, EP-570838, EP-796851, EP-775698 and EP-878469; benzophenone derivatives; benzalmalonate derivatives; benzimidazol, imidizoline derivatives; p-aminobenzoic acid derivatives; polymeric and silicone filters.

Appropiate inorganic filters include for instance metallic oxides and treated and untreated pigments, nanopigments, such as titanium dioxide (amorphous or crystalline), iron oxides, zinc, zirconium or cerium. In addition, alumina and/or aluminium stearate are conventional coating agents. Examples of untreated metallic oxides as (non-coated) inorganic filters are those described in patent applications EP518772 and EP518773.

The cosmetic, dermatological and pharmaceutical formulations of the present invention can additionally contain additives and adjuvants that can be selected from fatty acids, organic solvents, thickening agents, softening agents, antioxidants, opacifiers, stabilisers, emollients, hydroxy-acids, antifoaming agents, moisturizing agents, vitamins, fragrances, preservatives, surfactants, sequestering agents, polymers, propellants, acidifying or basifying agents, colorants, dyes, dihydroxyacetone, insect repellent or any other ingredient commonly used in cosmetic formulations, and in particular in the production of photoprotective compositions.

Examples of substances/fatty acids include, among others, oils or waxes or mixtures thereof and they can include fatty acids, fatty alcohols and fatty acid esters. The oils are selected, advantageously, from animal, vegetable or synthetic oils, and in particular from liquid petrolatum, liquid paraffin, volatile and non-volatile silicone oils, isoparaffins, polyalphaolefins, or fluorinated or perfluorinated oils. Similarly, the waxes are selected, advantageously, from animal, vegetable, mineral or synthetic waxes well known to the skilled in the art. Examples of organic solvents include short alcohols and polyols.

The thickeners are selected, advantageously, from acrylic-acid cross-linked polymers, modified and unmodified locust bean gums, celluloses and xanthan gums, such as hydroxypropylated locust bean gums, methylhydroxyethylcellulose, hydroxypropylmethylcellulose or hydroxyethylcellulose .

When selecting the excipients, adjuvants, etc., the skilled in the art will ensure that they do not affect the activity of the bis-resorcinyl triazine derivatives of general formula (I) in accordance with the invention.

Throughout the description and claims the word "comprise" and variations of the word, such as "comprising", are not intended to exclude other technical features, additives, components, or steps.

Furthermore, the present invention covers all possible combinations of particular and preferred groups described hereinabove.

Additional objects, advantages and features of the invention will become apparent to those skilled in the art upon examination of the description or may be learned by practice of the invention. The following examples and drawings are provided by way of illustration, and are not intended to be limiting of the present invention.

### BRIEF DESCRIPTION OF THE DRAWINGS

Figure 1.-
   A) UV profile of the compounds of examples 1 and 2.
   B) UV profile of the compounds of examples 3, 4, 5 and 6.
Figure 2.-
   A) UV profile of the compounds of examples 7, 8, 9 and 10.
   B) UV profile of the compounds of examples 11, 12, 13 and 14.
Figure 3.- Photo-stability in Miglyol 812 after 2 h irradiation of the compounds of Examples 1, 2, 4, 5, 6, 7, 12 and 14 compared with *bis*-ethylhexyloxyphenol methoxyphenyl triazine (BEMT) and *tert*-butyl methoxydibenzoyl methane (BMDBM).
Figure 4.- Photo-stability in Miglyol 812 after 4 h irradiation of compounds of Examples 1, 5, 8, 9, 12 and 14 compared with *bis*-ethylhexyloxyphenol methoxyphenyl triazine (BEMT) and *tert*-butyl methoxydibenzoyl methane (BMDBM).
Figure 5.- Photo-stability in Miglyol after 2 h irradiation of Example 7 in combination with with ethylhexyl methoxycinnamate (EHMC).
Figure 6.- Photo-stability in Miglyol after 4 h irradiation of Examples 1 and 12 in combination with *tert*-butyl methoxydibenzoyl methane (BMDBM).

### DETAILED DESCRIPTION OF PARTICULAR EMBODIMENTS

### Example 1.

### (1) Synthesis of 2-(1-methyl-pyrazol-5-yl)-4,6-bis[4-(2-ethylhexyloxy)-3-methyl-2-hydroxy-phenyl]-1,3,5-triazine.

### a) 2-(1-Methyl-pyrazol-5-yl)-4,6-bis(3-methyl-2,4-dihydroxyphenyl)-1,3,5-triazine.

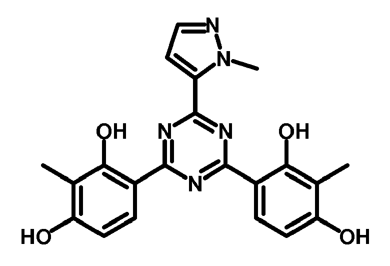

To a mixture of 2-methylresorcinol (4.27 g, 34.4 mmol) in 1,2-dichloroethane (200 mL) was added 2-(1-methyl-pyrazol-5-yl)-4,6-dichloro-1,3,5-triazine (3.96 g, 17.2 mmol) and aluminium trichloride (4.58 g, 34.4 mmol) and kept at 100°C for 16 hours. The mixture was cooled. HCl 2N (120 mL) was added and left stirring for 1 h. The solvent was removed under low pressure. Water (50 mL) was added and the solid obtained was filtered, washed with water (3 × 50 mL), and dried. 2-(1-Methyl-pyrazol-5-yl)-4,6-bis(3-methyl-2,4-dihydroxyphenyl)-1,3,5-triazine (3.33 g, 48 %) was obtained as yellow solid.
¹H-NMR (300 MHz, DMSO-d₆): δ 2.00 (s, 6H), 4.30 (s, 3H), 6.56 (d, J = 9.0 Hz, 1H), 7.05 (d, J= 2.1 Hz, 1H), 7.63 (d, J = 2.2 Hz, 1H), 7.91 (d, J = 8.8 Hz, 2H), 10.46 (s, 2H), 13.26 (m, 2H).

### b) 2-(1-Methyl-pyrazol-5-yl)-4,6-bis[4-(2-ethylhexyloxy)-3-methyl-2-hydroxyphenyl]-1,3,5-triazine.

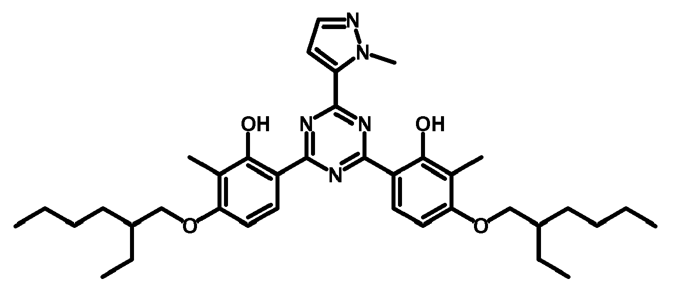

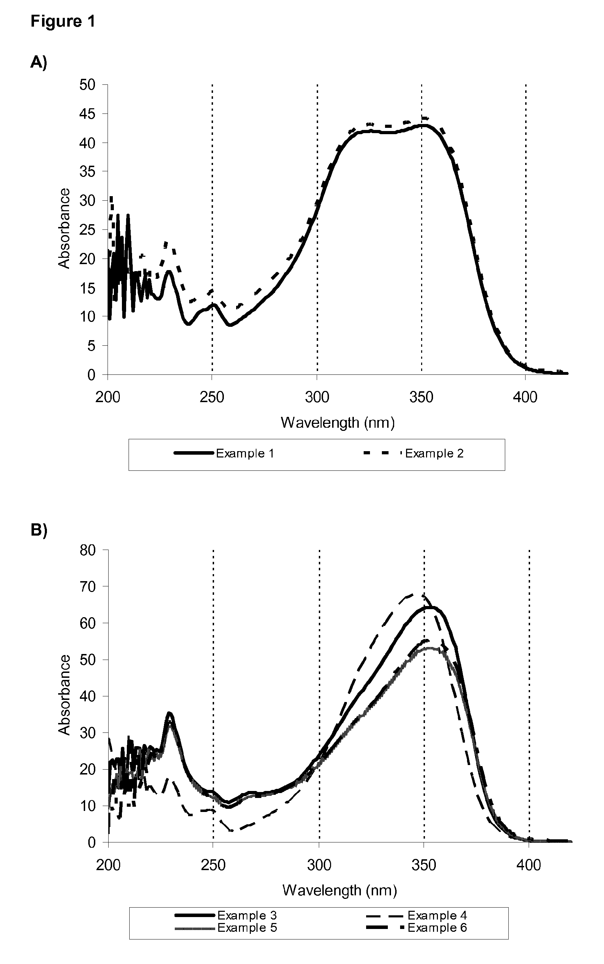

To a solution of 2-(1-methyl-pyrazol-5-yl)-4,6-bis(3-methyl-2,4-dihydroxyphenyl)-1,3,5-triazine (1.5 g, 3.7 mmol) in DMF (30 mL), Na₂CO₃ (1.29 g, 12.2 mmol) was added, and the mixture was heated to 70°C. A solution of 3-(bromomethyl)heptane (1.97 mL, 11.1 mmol) in DMF (20 mL) was added slowly. Once the addition was finished, the system was heated to 110°C for 16 hours. The mixture was cooled. The solvent was removed at reduced pressure and the residue was diluted in toluene/acetone 7/3 (100 mL). The organic phase was filtered to remove inorganic salts, and the solvent was eliminated at reduced pressure. The crude was suspended in MeOH (50 mL), stirred for 16 h., filtered, and washed with MeOH (3 × 10 mL). The solid was dried to furnish 2-(1-methyl-pyrazol-5-yl)-4,6-bis[4-(2-ethylhexyloxy)-3-methyl-2-hydroxy-phenyl]-1,3,5-triazine (1.4 g, 60 %), a yellowish solid.
¹H-NMR (300 MHz, CDCl₃): δ 0.96 (m, 12H), 1.35 (bs, 8H), 1.51 (m, 8H), 1.78 (m, 2H), 2.19 (s, 6H), 3.97 (d, J = 5.5 Hz, 4H), 4.49 (s, 3H), 6.59 (d, J = 9.0 Hz, 2H), 7.22 (d, J = 2.0 Hz, 1H), 7.60 (d, J = 2.0 Hz, 1H), 8.20 (m, 2H), 13.26 (m, 2H). UV: λₘₐₓ = 326 nm; εₘₐₓ = 43000 M⁻¹ cm⁻¹; λₘₐₓ = 352 nm; εₘₐₓ = 43000 M⁻¹ cm⁻¹ (CH₂Cl₂).
The UV profile and λₘₐₓ show a balanced absorption of UV radiation in both the UVB and UVA regions, making this compound especially interesting due to its large range in covering/absorbing the UV radiation arriving from the sun (see Fig. 1A).

### Example 2.

### (2) Synthesis of 2-(1-methyl-pyrazol-5-yl)-4,6-bis[4-(2-butyloctyloxy)-3-methyl-2-hydroxy-phenyl]-1,3,5-triazine.

### a) 2-(1-Methyl-pyrazol-5-yl)-4,6-bis[4-(2-butyloctyloxy)-3-methyl-2-hydroxyphenyl]-1,3,5-triazine.

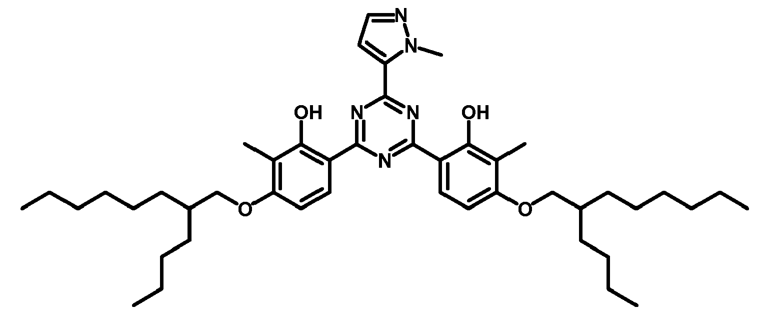

This compound was synthesized following the general procedure described above (see part 1b)) using 2-(1-methyl-pyrazol-5-yl)-4,6-bis(3-methyl-2,4-dihydroxyphenyl)-1,3,5-triazine and 5-(bromomethyl)undecane (20 % yield, yellowish solid).
¹H-NMR (300 MHz, CDCl₃): δ 0.90 (m, 12H), 1.32 (bs, 24H), 1.46 (m, 8H), 1.83 (m, 2H), 2.19 (s, 6H), 3.96 (d, J = 5.2 Hz, 4H), 4.48 (s, 3H), 6.59 (d, J = 9.1 Hz, 2H), 7.22 (d, J = 2.0 Hz, 1H), 7.60 (m, 1H), 8.21 (m, 2H), 13.30 (m, 2H). UV: λₘₐₓ = 326 nm; εₘₐₓ = 43000 M⁻¹ cm⁻¹; λₘₐₓ = 352 nm; εₘₐₓ = 44000 M⁻¹ cm⁻¹ (CH₂Cl₂).
The UV profile and λₘₐₓ show a balanced absorption of UV radiation in both the UVB and UVA regions, making this compound especially interesting due to its large range in covering/absorbing the UV radiation arriving from the sun (see Fig. 1A).

### Example 3.

### (3) Synthesis of 6,6'-(6-(1-methyl-1H-indol-3-yl)-1,3,5-triazine-2,4-diyl)bis(3-(2-ethylhexyloxy)-2-methylphenol).

### a) 3-(4,6-Dichloro-1,3,5-triazin-2-yl)-1-methyl-1H-indole.

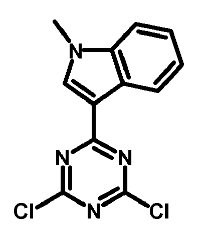

A mixture of 1-methylindole (5.0 mL, 49.6 mmol) and 2,4,6-trichloro-1,3,5-triazine (8.9 g, 48.6 mmol) was refluxed in xylene (35 mL) for 16 hours. The solvent was evaporated to dryness; acetonitrile (35 mL) was added at 0°C, and the mixture was left under stirring for 25 min. The solid obtained was filtered, washed with cold acetonitrile (2 × 10 mL) and dried to obtain 3-(4,6-dichloro-1,3,5-triazin-2-yl)-1-methyl-1H-indole (2.84 g, 27.4 mmol, 21 %), as yellow solid.

¹H-NMR (300 MHz, CDCl₃): δ 3.92 (s, 3H), 7.40 (m, 3H), 8.31 (s, 1H), 8.54 (m, 1H).

### b) 4,4'-(6-(1-Methyl-1H-indol-3-yl)-1,3,5-triazine-2,4-diyl)bis(2-methylbenzene-1,3-diol).

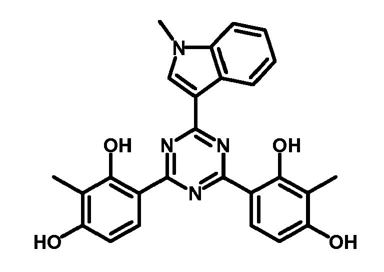

This compound was synthesized following the general procedure described above (see part 1a)) using 3-(4,6-dichloro-1,3,5-triazin-2-yl)-1-methyl-1H-indole (94 % yield, yellow solid).

¹H-NMR (300 MHz, CDCl₃): δ 2.07 (s, 6H), 3.98 (s, 3H), 6.62 (d, J= 8.8 Hz, 2H), 7.36 (m, 3H), 7.62 (m, 1H), 7.37 (d, J = 8.8 Hz, 1H), 8.51 (s, 1H), 10.36 (s, 2H), 13.83 (s, 2H).

### c) 6,6'-(6-(1-Methyl-1H-indol-3-yl)-1,3,5-triazine-2,4-diyl)bis(3-(2-ethylhexyloxy)-2-methylphenol).

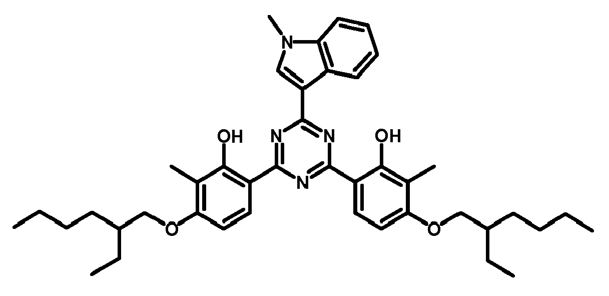

This compound was synthesized following the general procedure described above (see part 1 b)) purifying by column chromatography (33 % yield, yellowish solid).
¹H-NMR (300 MHz, CDCl₃): δ 0.96 (m, 12H), 1.37 (m, 8H), 1.53 (m, 8H), 1.81 (m, 2H), 2.24 (s, 6H), 3.95 (s, 3H), 4.00 (d, J = 5.5 Hz, 4H), 6.64 (d, J = 9.1 Hz, 2H), 7.42 (m, 4H), 8.19 (s, 1H), 8.39 (m, 2H), 8.67 (m, 2H). UV: λₘₐₛ = 354 nm; εₘₐₓ = 63000 M⁻¹ cm⁻¹ (CH₂Cl₂).
The UV profile and λₘₐₓ show a boosted absorption of UV radiation in the UVA region, together with a remarkable absorption in the UVB region which lacks in most of UVA absorbers. Furthermore, its power of absorption almost doubles those of the UVA absorbers in the market (see Fig. 1B).

### Example 4.

### (4) Synthesis of 6,6'-(6-(1-methyl-1H-indol-3-yl)-1,3,5-triazine-2,4-diyl)bis(3-(2-butyloctyloxy)-2-methylphenol).

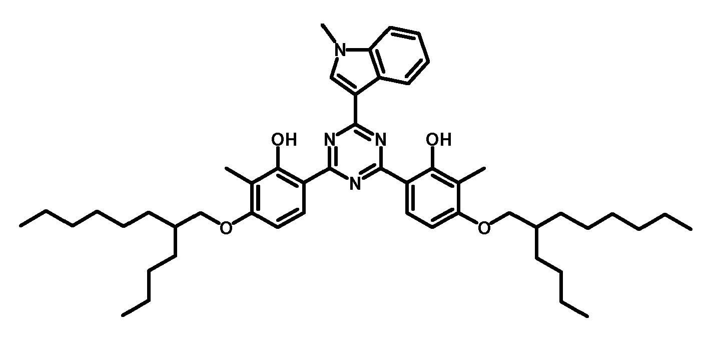

This compound was synthesized following the general procedure described above (see part 1b)) using 5-(bromomethyl)undecane and purifying by column chromatography (63 % yield, yellowish oil).

¹H-NMR (300 MHz, CDCl₃): δ 0.91 (m, 12H), 1.32 (bs, 32H), 1.84 (m, 2H), 2.23 (s, 6H), 3.92 (s, 3H), 3.97 (d, J = 5.5 Hz, 4H), 6.61 (d, J = 9.1 Hz, 2H), 7.39 (m, 4H), 8.15 (s, 1H), 8.65 (m, 2H). UV: λₘₐₓ = 353 nm; εₘₐₓ = 64000 M⁻¹ cm⁻¹ (CH₂Cl₂).
The UV profile and λₘₐₓ show a boosted absorption of UV radiation in the UVA region, together with a remarkable absorption in the UVB region which lacks in most of UVA absorbers. Furthermore, its power of absorption almost doubles those of the UVA absorbers in the market (see Fig. 1B).

### Example 5.

### (5) Synthesis of 6,6'-(6-(1-(2-ethylhexyl)-1H-indol-3-yl)-1,3,5-triazine-2,4-diyl)bis(3-(2-ethylhexyloxy)-2-methylphenol).

### a) 1-(2-ethylhexyl)-1H-indole.

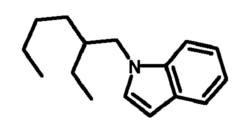

Indole (5 g, 42.68 mmol, 1 equiv.) and NaH 60 % (2.56 g, 64.92 mmol, 1.5 equiv.) were introduced in a round bottom flask under N₂ atmosphere. DMF anh (100 mL) was added dropwise at 0°C and stirred until H₂ evolution had finished. 3-(Bromomethyl)heptane (7.59 mL, 42.68 mmol, 1 equiv.) was added dropwise and stirred at r.t. for 16 hours. The crude mixture was filtered and the solvent was evaporated. The residue was dissolved in petroleum ether (50 mL), filtered and the solvent was evaporated, obtaining 7.6 g (79 % yield, yellow oil).

¹H-NMR (300 MHz, CDCl₃): δ 0.90 (m, 6H), 1.30 (m, 8H), 1.91 (m, 1H), 4.00 (m, 2H), 6.49 (dd, J = 3.0, 0.8 Hz, 1H), 7.08 (dm, J = 3.0 Hz, 1H), 7.10 (m, 1H), 7.20 (dt, J = 7.7 Hz, 1H), 7.34 (dm, J = 8.2 Hz, 1H), 7.63 (dm, J = 7.7 Hz, 1H).

### b) 3-(4,6-Dichloro-1,3,5-triazin-2-yl)-1-(2-ethylhexyl)-1H-indole.

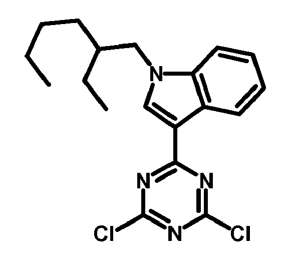

This compound was synthesized following the general procedure described above (see part (3)a)) using 1-(2-ethylhexyl)-1H-indole (99 % yield, brown oil).

¹H-NMR (300 MHz, CDCl₃): δ 0.90 (m, 6H), 1.32 (m, 8H), 1.96 (m, 1H), 4.07 (m, 2H), 7.38 (bs, 3H), 8.28 (s, 1H), 8.55 (m, 1H).

### c) 4,4'-(6-(1-(2-Ethylhexyl)-1H-indol-3-yl)-1,3,5-triazine-2,4-diyl)bis(2-methylbenzene-1,3-diol).

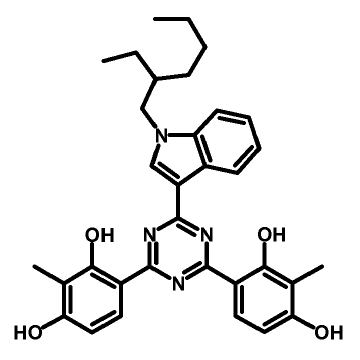

This compound was synthesized following the general procedure described above (see part 1a)) using 3-(4,6-dichloro-1,3,5-triazin-2-yl)-1-(2-ethylhexyl)-1H-indole (80 % yield, orange solid).

¹H-NMR (300 MHz, CDCl₃): δ 0.83 (m, 6H), 1.24 (m, 8H), 1.92 (m, 1H), 2.07 (s, 6H), 4.27 (d, J = 7.2 Hz, 2H), 6.62 (d, J= 9 Hz, 2H), 7.35 (m, 3H), 7.62 (m, 1H), 8.10 (d, J = 9 Hz, 2H), 8.44 (d, J = 8 Hz, 1H), 8.47 (s, 1H), 10.35 (s, 2H), 13.80 (s, 2H).

### d) 6,6'-(6-(1-(2-Ethylhexyl)-1H-indol-3-yl)-1,3,5-triazine-2,4-diyl)bis(3-(2-ethylhexyloxy)-2-methylphenol).

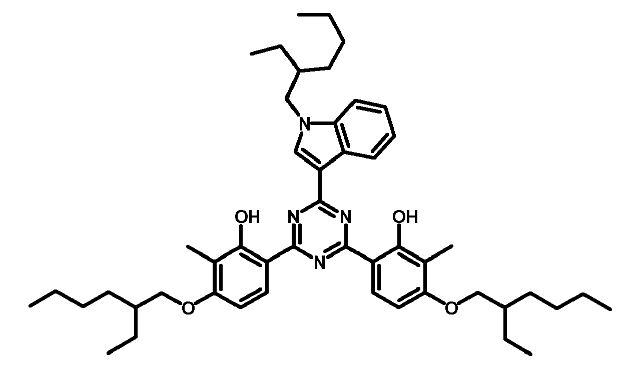

This compound was synthesized following the general procedure described above (see part 1b)) using 4,4'-(6-(1-(2-ethylhexyl)-1H-indol-3-yl)-1,3,5-triazine-2,4-diyl)bis(2-methylbenzene-1,3-diol) and purifying by column chromatography (29 % yield, yellowish oil).

¹H-NMR (300 MHz, CDCl₃): δ 0.96 (m, 18H), 1.37 (m, 12H), 1.53 (m, 12H), 1.79 (m, 2H), 2.01 (m, 1H), 2.24 (s, 6H), 3.99 (d, J = 5.5 Hz, 4H), 4.11 (m, 2H), 6.63 (d, J = 9 Hz, 2H), 7.38 (m, 4H), 8.13 (s, 1H), 8.37 (m, 1H), 8.67 (m, 1H), 13.91 (s, 2H). UV: λₘₐₓ = 354 nm; εₘₐₓ = 54000 M⁻¹ cm⁻¹ (CH₂Cl₂).
The UV profile and λₘₐₓ show a boosted absorption of UV radiation in the UVA region, together with a remarkable absorption in the UVB region which lacks in most of UVA absorbers. Furthermore, its power of absorption almost doubles those of the UVA absorbers in the market (see Fig. 1B).

### Example 6.

### (6) Synthesis of 6,6'-(6-(1-(2-ethylhexyl)-1H-indol-3-yl)-1,3,5-triazine-2,4-diyl)bis(3-(2-butyloctyloxy)-2-methylphenol).

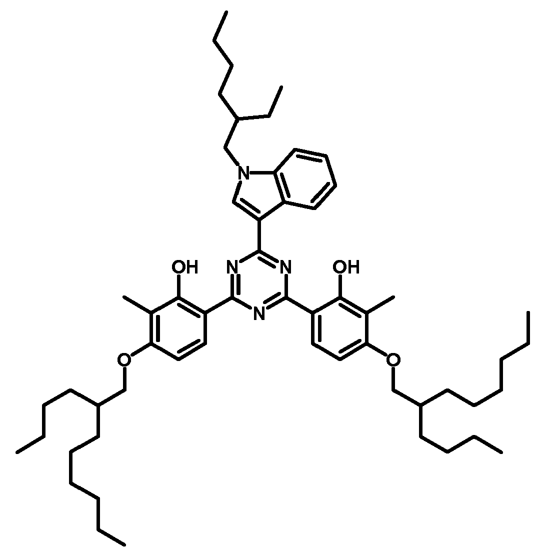

This compound was synthesized following the general procedure described above (see part 1b)) using 5-(bromomethyl)undecane and purifying by column chromatography (65 % yield, yellowish oil).

¹H-NMR (300 MHz, CDCl₃): δ 0.92 (m, 18H), 1.36 (m, 40H), 1.85 (m, 2H), 1.99 (m, 1H), 2.24 (s, 6H), 3.97 (d, J = 5.5 Hz, 4H), 4.10 (m, 2H), 6.62 (d, J = 9 Hz, 2H), 7.38 (m, 4H), 8.13 (s, 1H), 8.36 (m, 1H), 8.68 (m, 1H), 13.91 (s, 2H).
UV: λₘₐₓ = 354 nm; εₘₐₓ = 54000 M⁻¹ cm⁻¹ (CH₂Cl₂).

The UV profile and λₘₐₓ show a boosted absorption of UV radiation in the UVA region, together with a remarkable absorption in the UVB region which lacks in most of UVA absorbers. Furthermore, its power of absorption almost doubles those of the UVA absorbers in the market (see Fig. 1B).

### Example 7.

### (7) Synthesis of 6,6'-(6-(1-(2-ethylhexyl)-5-methoxy-1H-indol-3-yl)-1,3,5-triazine-2,4-diyl)bis(3-(2-ethylhexyloxy)-2-methylphenol).

### a) 1-(2-ethylhexyl)-5-methoxy-1H-indole.

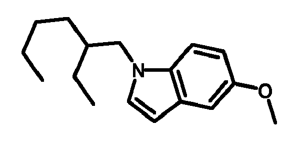

This compound was synthesized following the general procedure described above (see part (5)a)) using 5-methoxy-1H-indole (90 % yield, yellow oil). ¹H-NMR (300 MHz, CDCl₃): δ 0.88 (m, 6H), 1.28 (m, 8H), 1.87 (m, 1H), 3.85 (s, 3H), 3.95 (m, 2H), 6.39 (dd, J = 3.0, 1 Hz, 1H), 6.86 (dd, J = 9, 2.5 Hz, 1H), 7.04 (d, J = 3 Hz, 1H), 7.09 (d, J = 2.5 Hz, 1H), 7.21 (d, J = 9 Hz, 1H), 8.02 (s, 1H).

### b) 3-(4,6-Dichloro-1,3,5-triazin-2-yl)-1-(2-ethylhexyl)-5-methoxy-1H-indole.

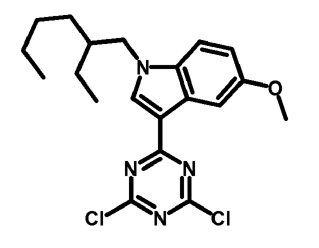

This compound was synthesized following the general procedure described above (see part (3)a)) using 1-(2-ethylhexyl)-5-methoxy-1H-indole (11 % yield, yellow oil).

¹H-NMR (300 MHz, CDCl₃): δ 0.90 (m, 6H), 1.30 (m, 8H), 1.94 (m, 1H), 3.94 (s, 3H), 4.03 (m, 2H), 6.97 (dd, J = 9, 2.5 Hz, 1H), 7.29 (s, 1H), 8.04 (d, J = 2.5 Hz, 1H), 8.23 (m, 1H).

### c) 4,4'-(6-(1-(2-Ethylhexyl)-5-methoxy-1H-indol-3-yl)-1,3,5-triazine-2,4-diyl)bis(2-methylbenzene-1,3-diol).

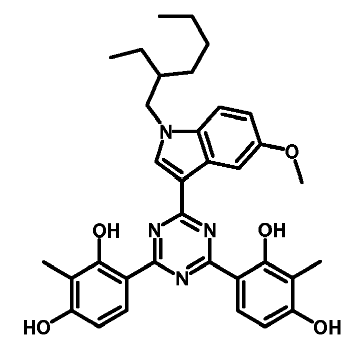

This compound was synthesized following the general procedure described above (see part 1a)) using 3-(4,6-dichloro-1,3,5-triazin-2-yl)-1-(2-ethylhexyl)-5-methoxy-1H-indole (64 % yield, yellow solid).

¹H-NMR (300 MHz, CDCl₃): δ 0.89 (m, 6H), 1.27 (m, 8H), 1.82 (m, 1H), 2.11 (s, 6H), 3.89 (s, 3H), 4.09 (m, 2H), 6.42 (d, J= 9 Hz, 2H), 6.79 (dd, J= 9, 2.5 Hz, 2H), 7.15 (d, J = 8.8 Hz, 1H), 7.81 (m, 1H), 7.83 (m, 1H), 7.97 (m, 2H).

### d) 6,6'-(6-(1-(2-Ethylhexyl)-5-methoxy-1H-indol-3-yl)-1,3,5-triazine-2,4-diyl)bis(3-(2-ethylhexyloxy)-2-methylphenol).

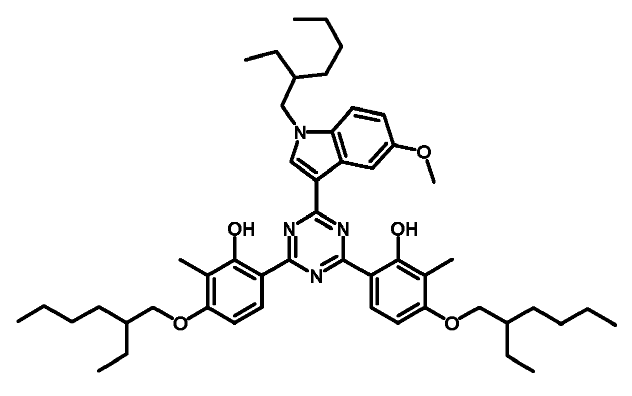

This compound was synthesized following the general procedure described above (see part 1b)) using 4,4'-(6-(1-(2-ethylhexyl)-5-methoxy-1H-indol-3-yl)-1,3,5-triazine-2,4-diyl)bis(2-methylbenzene-1,3-diol) and purifying by column chromatography (54 % yield, yellowish oil).

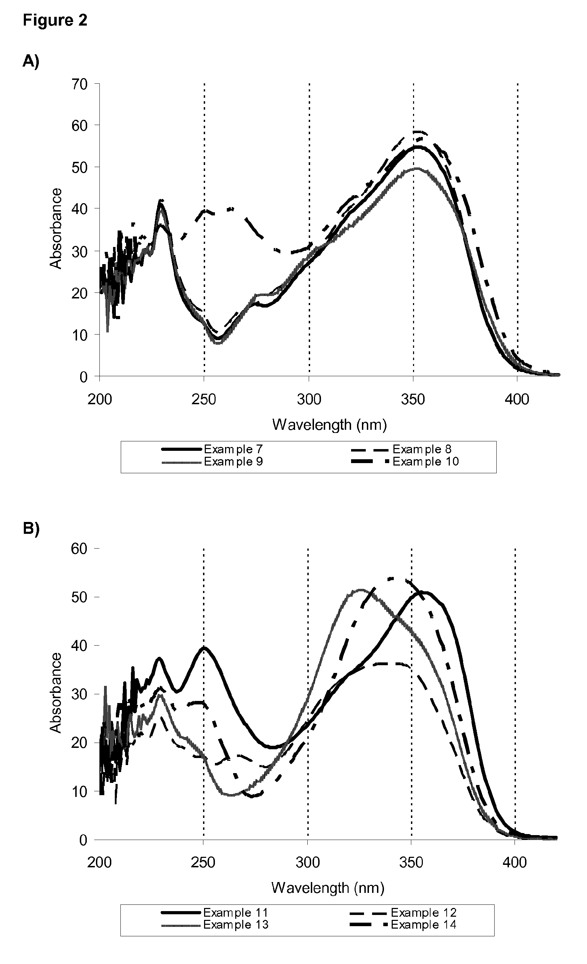

¹H-NMR (300 MHz, CDCl₃): δ 0.95 (m, 18H), 1.37 (m, 16H), 1.53 (m, 8H), 1.79 (m, 2H), 1.95 (m, 1H), 2.21 (s, 6H), 3.98 (d, J = 5.5 Hz, 4H), 4.01 (s, 3H), 4.03 (m, 2H), 6.58 (d, J = 9 Hz, 2H), 6.96 (dd, J = 9, 2.5 Hz, 1H), 7.26 (d, J = 9 Hz, 1H), 8.05 (s, 1H), 8.13 (bs, 2H), 8.33 (bs, 1H), 13.93 (s, 2H).
UV: λₘₐₓ = 353 nm; εₘₐₓ = 54000 M⁻¹ cm⁻¹ (CH₂Cl₂).
The UV profile and λₘₐₓ show a boosted absorption of UV radiation in the UVA region, together with a remarkable absorption in the UVB region which lacks in most of UVA absorbers. Furthermore, its power of absorption is significantly greater to those of the UVA absorbers in the market. The substitution in the heterocyclic ring allows a better covering in the UVB region, without any loss in covering the UVA region (see Fig. 2A).

### Example 8.

### (8) Synthesis of 6,6'-(6-(1-(2-ethylhexyl)-2-methyl-1H-indol-3-yl)-1,3,5-triazine-2,4-diyl)bis(3-(2-ethylhexyloxy)-2-methylphenol).

### a) 1-(2-ethylhexyl)-2-methyl-1H-indole.

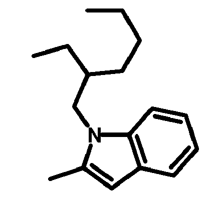

This compound was synthesized following the general procedure described above (see part (5)a)) using 2-methyl-1H-indole (41 % yield, yellow oil).

¹H-NMR (300 MHz, CDCl₃): δ 0.88 (m, 6H), 1.30 (m, 8H), 1.90 (m, 1H), 2.42 (s, 3H), 3.93 (m, 2H), 6.23 (m, 1H), 7.04 (m, 1H), 7.11 (m, 1H), 7.23 (m, 1H), 7.51 (dm, J 7.7 Hz, 1H).

### b) 3-(4,6-Dichloro-1,3,5-triazin-2-yl)-1-(2-ethylhexyl)-2-methyl-1H-indole.

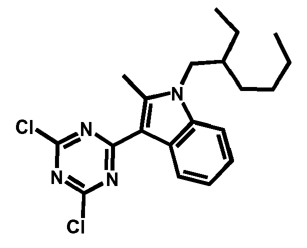

This compound was synthesized following the general procedure described above (see part (3)a)) using 1-(2-ethylhexyl)-2-methyl-1H-indole (99 % yield, brown oil).

¹H-NMR (300 MHz, CDCl₃): δ 0.90 (m, 6H), 1.30 (m, 8H), 1.95 (m, 1H), 2.96 (s, 3H), 4.08 (d, J = 7.7 Hz, 2H), 7.32 (m, 3H), 8.63 (m, 1H).

### c) 4,4'-(6-(1-(2-Ethylhexyl)-2-methyl-1H-indol-3-yl)-1,3,5-triazine-2,4-diyl)bis(2-methylbenzene-1,3-diol).

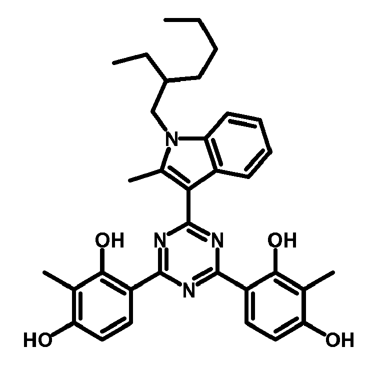

This compound was synthesized following the general procedure described above (see part 1a)) using 3-(4,6-dichloro-1,3,5-triazin-2-yl)-1-(2-ethylhexyl)-2-methyl-1H-indole (84% yield, yellow solid).

¹H-NMR (300 MHz, CDCl₃): δ 0.84 (m, 6H), 1.24 (m, 8H), 1.88 (m, 1H), 2.05 (s, 6H), 2.91 (s, 3H), 4.20 (d, J =7.7 Hz, 2H), 6.61 (d, J= 9 Hz, 2H), 7.26 (m, 2H), 7.58 (m, 1H), 8.08 (d, J = 9 Hz, 2H), 8.29 (m, 1H), 10.34 (s, 2H), 13.72 (s, 2H).

### d) 6,6'-(6-(1-(2-Ethylhexyl)-2-methyl-1H-indol-3-yl)-1,3,5-triazine-2,4-diyl)bis(3-(2-ethylhexyloxy)-2-methylphenol).

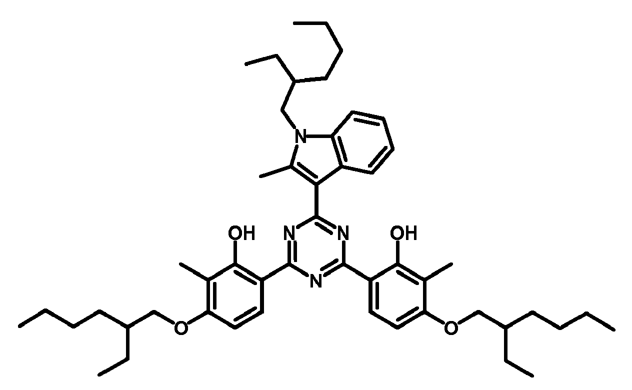

This compound was synthesized following the general procedure described above (see part 1b)) using 4,4'-(6-(1-(2-ethylhexyl)-2-methyl-1H-indol-3-yl)-1,3,5-triazine-2,4-diyl)bis(2-methylbenzene-1,3-diol) and purifying by column chromatography (40 % yield, yellow oil).
¹H-NMR (300 MHz, CDCl₃): δ 0.94 (m, 18H), 1.36 (m, 16H), 1.50 (m, 8H), 1.79 (m, 2H), 1.96 (m, 1H), 2.22 (s, 6H), 2.95 (s, 3H), 3.98 (d, J = 5.5 Hz, 4H), 4.08 (m, 2H), 6.61 (d, J = 9 Hz, 2H), 7.30 (m, 1H), 7.35 (m, 2H), 8.34 (bs, 2H), 8.51 (bs, 1H), 13.71 (s, 2H). UV: λₘₐₓ = 352 nm; εₘₐₓ = 58000 M⁻¹ cm⁻¹ (CH₂Cl₂).
The UV profile and λₘₐₓ show a boosted absorption of UV radiation in the UVA region, together with a remarkable absorption in the UVB region which lacks in most of UVA absorbers. Furthermore, its power of absorption is significantly greater to those of the UVA absorbers in the market. The substitution in the heterocyclic ring allows a better covering in the UVB region, without any loss in covering the UVA region (see Fig. 2A).

### Example 9.

### (9) Synthesis of 6,6'-(6-(1-(2-ethylhexyl)-5-methoxy-2-methyl-1H-indol-3-yl)-1,3,5-triazine-2,4-diyl)bis(3-(2-ethylhexyloxy)-2-methylphenol).

### a) 1-(2-ethylhexyl)-5-methoxy-2-methyl-1H-indole.

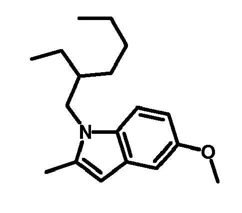

This compound was synthesized following the general procedure described above (see part (5)a)) using 5-methoxy-2-methyl-1H-indole (36 % yield, colourless oil).
¹H-NMR (300 MHz, CDCl₃): δ 0.88 (m, 6H), 1.28 (m, 8H), 1.86 (m, 1H), 2.39 (s, 3H), 3.84 (s, 3H), 3.89 (m, 2H), 6.15 (s, 1H), 6.77 (dd, J = 9, 2.5 Hz, 1H), 6.99 (d, J = 2.5 Hz, 1H), 7.13 (d, J = 9 Hz, 1H).

### b) 3-(4,6-Dichloro-1,3,5-triazin-2-yl)-1-(2-ethylhexyl)-5-methoxy-2-methyl-1H-indole.

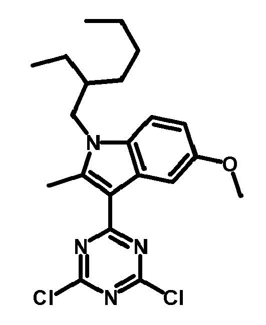

This compound was synthesized following the general procedure described above (see part (3)a)) using 1-(2-ethylhexyl)-5-methoxy-2-methyl-1H-indole (99 % yield, orange oil).
¹H-NMR (300 MHz, CDCl₃): δ 0.81 (m, 6H), 1.21 (m, 8H), 1.83 (m, 1H), 2.86 (s, 3H), 3.85 (s, 3H), 3.97 (m, 2H), 6.83 (dd, J = 9, 2.5 Hz, 1H), 7.15 (d, J = 9 Hz, 1H), 8.09 (d, J = 2.5 Hz, 1H).

### c) 4,4'-(6-(1-(2-Ethylhexyl)-5-methoxy-2-methyl-1H-indol-3-yl)-1,3,5-triazine-2,4-diyl)bis(2-methylbenzene-1,3-diol).

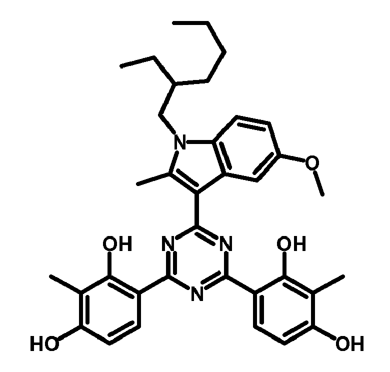

This compound was synthesized following the general procedure described above (see part 1a)) using 3-(4,6-dichloro-1,3,5-triazin-2-yl)-1-(2-ethylhexyl)-5-methoxy-2-methyl-1H-indole (29 % yield, orange solid).
¹H-NMR (300 MHz, CDCl₃): δ 0.84 (m, 6H), 1.23 (m, 8H), 1.83 (m, 1H), 2.06 (s, 6H), 2.86 (s, 3H), 3.85 (s, 3H), 4.11 (d, J = 7.5 Hz, 2H), 6.60 (d, J = 9 Hz, 2H), 6.86 (dd, J = 9, 2.5 Hz, 1H), 7.43 (d, J = 9 Hz, 1H), 7.84 (d, J = 2.5 Hz, 1H), 8.07 (d, J = 9 Hz, 2H), 10.30 (s, 2H), 13.79 (s, 2H).

### d) 6,6'-(6-(1-(2-Ethylhexyl)-5-methoxy-2-methyl-1H-indol-3-yl)-1,3,5-triazine-2,4-diyl)bis(3-(2-ethylhexyloxy)-2-methylphenol).

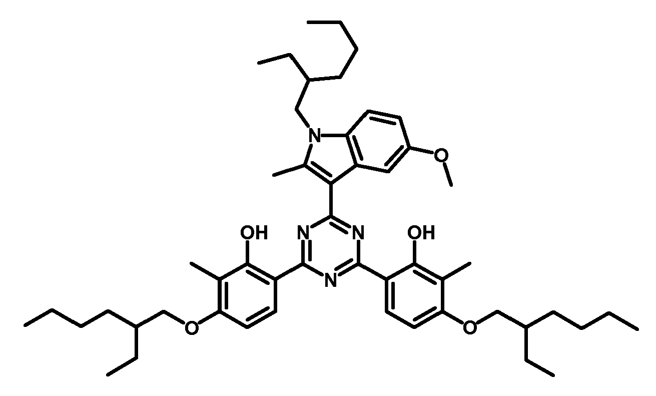

This compound was synthesized following the general procedure described above (see part 1b)) using 4,4'-(6-(1-(2-ethylhexyl)-5-methoxy-2-methyl-1H-indol-3-yl)-1,3,5-triazine-2,4-diyl)bis(2-methylbenzene-1,3-diol) and purifying by column chromatography (65 % yield, yellowish oil).

¹H-NMR (300 MHz, CDCl₃): δ 0.94 (m, 18H), 1.35 (m, 16H), 1.51 (m, 8H), 1.79 (m, 2H), 1.94 (m, 1H), 2.20 (s, 6H), 2.95 (s, 3H), 3.97 (s, 3H), 4.05 (m, 2H), 6.59 (d, J = 9 Hz, 2H), 6.92 (dd, J = 9, 2.5 Hz, 1H), 7.24 (d, J = 9 Hz, 1H), 8.03 (s, 1H), 8.34 (bs, 2H), 13.81 (s, 2H). UV: λₘₐₓ = 351 nm; εₘₐₓ = 50000 M⁻¹ cm⁻¹ (CH₂Cl₂).
The UV profile and λₘₐₓ show a boosted absorption of UV radiation in the UVA region, together with a remarkable absorption in the UVB region which lacks in most of UVA absorbers. Furthermore, its power of absorption is significantly greater to those of the UVA absorbers in the market. The substitution in the heterocyclic ring allows a better covering in the UVB region, without any loss in covering the UVA region (see Fig. 2A).

### Example 10.

### (10) Synthesis of 6,6'-(6-(1-(2-ethylhexyl)-5-(4-methoxyphenyl)-1H-indol-3-yl)-1,3,5-triazine-2,4-diyl)bis(3-(2-ethylhexyloxy)-2-methylphenol).

### a) 5-bromo-1-(2-ethylhexyl)-1H-indole.

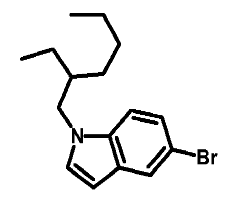

This compound was synthesized following the general procedure described above (see part (5)a)) using 5-bromo-1H-indole (89 % yield, yellowish oil).

¹H-NMR (300 MHz, CDCl₃): δ 0.88 (m, 6H), 1.27 (m, 8H), 1.85 (m, 1H), 3.96 (m, 2H), 7.06 (d, J = 3 Hz, 1H), 7.19 (m, 1H), 7.25 (m, 1H), 7.27 (m, 1H), 7.74 (d, J = 2 Hz, 1H).

### b) 1-(2-Ethylhexyl)-5-(4-methoxyphenyl)-1H-indole.

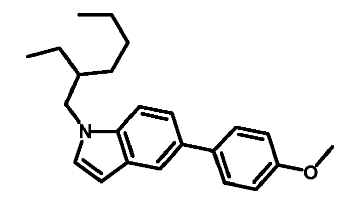

Under Ar atmosphere, 5-bromo-1-(2-ethylhexyl)-1H-indole (2 g, 6,48 mmol, 1 equiv.) and 4-methoxyphenylboronic acid (1,96 g, 12,96 mmol, 2 equiv.) were dissolved in a toluene/EtOH 1/1 mixture (20 mL). Na₂CO₃ 1 M solution (15,3 mL, 2,35 equiv.) and a solution of Pd(PPh₃)₄ (134 mg, 0,13 mmol, 0,02 equiv.) in a toluene/EtOH 1/1 mixture (20 mL) were added. The reaction mixture was heated to reflux for 1 h and additional Pd(PPh₃)₄ (134 mg, 0,13 mmol, 0,02 equiv.) was added, keeping refluxing for 16 h. Once the system was cooled, the solvent was removed. Petroleum ether was added (20 mL), the system was stirred and the organic layer was separated. The process was repeated 3 times. The whole organic layer was evaporated *in vacuo*, yielding 1-(2-ethylhexyl)-5-(4-methoxyphenyl)-1H-indole (76 % yield, off-white wax).
¹H-NMR (300 MHz, CDCl₃): δ 0.92 (m, 6H), 1.32 (m, 8H), 1.94 (m, 1H), 3.87 (s, 3H), 4.01 (m, 2H), 6.52 (dm, J = 3 Hz, 1H), 6.99 (m, 2H), 7.10 (d, J = 3 Hz, 1H), 7.37 (m, 1H), 7.42 (m, 1H), 7.59 (dm, J = 9 Hz, 2H), 7.79 (m, 1H).

### c) 3-(4,6-Dichloro-1,3,5-triazin-2-yl)-1-(2-ethylhexyl)-5-(4-methoxyphenyl)-1H-indole.

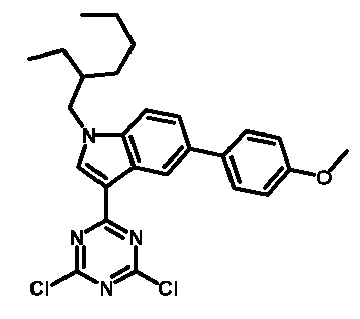

This compound was synthesized following the general procedure described above (see part (3)a)) using 1-(2-ethylhexyl)-5-(4-methoxyphenyl)-1H-indole (23 % yield, pink oil).

¹H-NMR (300 MHz, CDCl₃): δ 0.92 (m, 6H), 1.34 (m, 8H), 2.00 (m, 1H), 3.89 (s, 3H), 4.10 (m, 2H), 7.05 (dm, J = 9 Hz, 2H), 7.43 (m, 1H), 7.56 (m, 1H), 7.66 (dm, J = 9 Hz, 2H), 8.31 (s, 1H), 8.73 (m, 1H).

### d) 4,4'-(6-(1-(2-Ethylhexyl)-5-(4-methoxyphenyl)-1H-indol-3-yl)-1,3,5-triazine-2,4-diyl)bis(2-methylbenzene-1,3-diol).

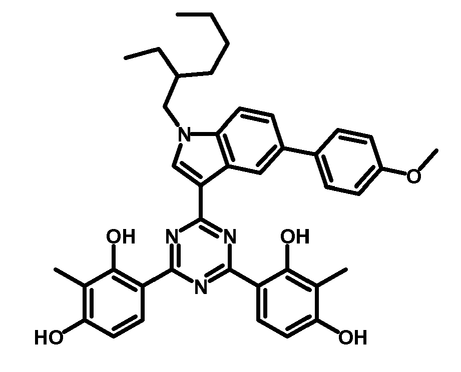

This compound was synthesized following the general procedure described above (see part 1a)) using 3-(4,6-dichloro-1,3,5-triazin-2-yl)-1-(2-ethylhexyl)-5-(4-methoxyphenyl)-1H-indole and purifying by column chromatography (39 % yield, yellow solid).
¹H-NMR (300 MHz, CDCl₃): δ 0.86 (m, 6H), 1.27 (m, 8H), 1.97 (m, 1H), 2.11 (s, 6H), 3.83 (s, 3H), 4.29 (d, J = 7.5 Hz, 2H), 6.62 (d, J = 9 Hz, 2H), 7.07 (d, J = 9 Hz, 2H), 7.62 (m, 1H), 7.68 (m, 1H), 7.81 (d, J = 9 Hz, 2H), 8.15 (d, J = 9 Hz, 2H), 8.54 (s, 1H), 8.69 (s, 1H), 10.37 (s, 2H), 13.91 (s, 2H).

### e) 6,6'-(6-(1-(2-Ethylhexyl)-5-(4-methoxyphenyl)-1H-indol-3-yl)-1,3,5-triazine-2,4-diyl)bis(3-(2-ethylhexyloxy)-2-methylphenol).

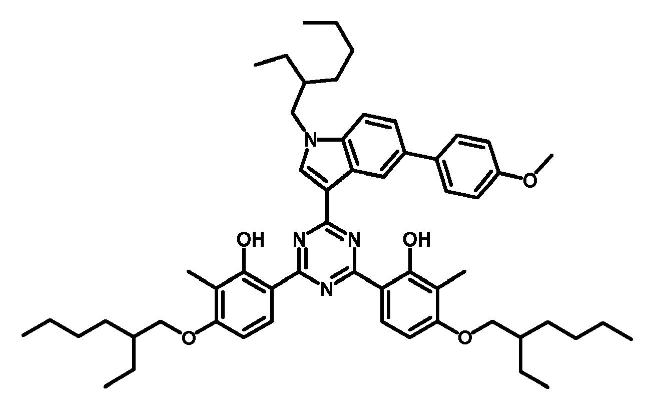

This compound was synthesized following the general procedure described above (see part 1b)) using 4,4'-(6-(1-(2-ethylhexyl)-5-(4-methoxyphenyl)-1H-indol-3-yl)-1,3,5-triazine-2,4-diyl)bis(2-methylbenzene-1,3-diol) and purifying by column chromatography (68 % yield, yellowish wax).

¹H-NMR (300 MHz, CDCl₃): δ 0.95 (m, 18H), 1.36 (m, 16H), 1.52 (m, 8H), 1.79 (m, 2H), 2.00 (m, 1H), 2.24 (s, 6H), 3.90 (s, 3H), 3.98 (d, J = 5.5 Hz, 4H), 4.08 (m, 2H), 6.58 (d, J = 9 Hz, 2H), 7.05 (d, J = 9 Hz, 2H), 7.40 (m, 1H), 7.56 (m, 1H), 7.81 (d, J = 9 Hz, 2H), 8.11 (s, 1H), 8.43 (bs, 2H), 8.86 (bs, 1H), 13.96 (s, 2H). UV: λₘₐₛ = 355 nm; εₘₐₓ = 57000 M⁻¹ cm⁻¹ (CH₂Cl₂).
The UV profile and λₘₐₓ show a boosted absorption of UV radiation in the UVA region, together with a remarkable absorption in the UVB region which lacks in most of UVA absorbers. Furthermore, its power of absorption is significantly greater to those of the UVA absorbers in the market. The substitution in the heterocyclic ring allows a better covering in the UVB region, without any loss in covering the UVA region (see Fig. 2A).

### Example 11.

### (11) Synthesis of 6,6'-(6-(1-(2-ethylhexyl)-5-phenyl-1H-indol-3-yl)-1,3,5-triazine-2,4-diyl)bis(3-(2-ethylhexyloxy)-2-methylphenol).

### a) 1-(2-ethylhexyl)-5-phenyl-1H--indole.

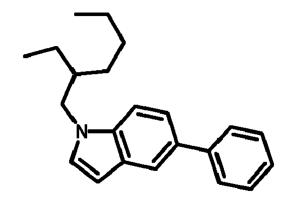

This compound was synthesized following the general procedure described above (see part (10)b)) using 5-bromo-1H-indole and phenylboronic acid (79 % yield, brown oil).

¹H-NMR (300 MHz, CDCl₃): δ 0.90 (m, 6H), 1.30 (m, 8H), 1.92 (m, 1H), 4.01 (m, 2H), 6.53 (m, 1H), 7.10 (m, 1H), 7.44 (m, 5H), 7.65 (d, J = 8 Hz, 2H), 7.84 (s, 1H).

### b) 3-(4,6-Dichloro-1,3,5-triazin-2-yl)-1-(2-ethylhexyl)-5-phenyl-1H-indole.

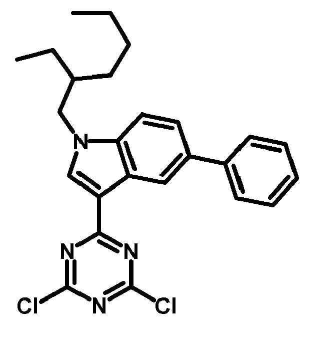

This compound was synthesized following the general procedure described above (see part (3)a)) using 1-(2-ethylhexyl)-5-phenyl-1H-indole (22 % yield, yellow solid).
¹H-NMR (300 MHz, CDCl₃): δ 0.91 (m, 6H), 1.32 (m, 8H), 2.00 (m, 1H), 4.09 (m, 2H), 7.39 (m, 1H), 7.49 (m, 3H), 7.60 (m, 1H), 7.72 (dm, J = 8 Hz, 2H), 8.32 (bs, 1H), 8.78 (bs, 1H).

### c) 4,4'-(6-(1-(2-Ethylhexyl)-5-phenyl-1H-indol-3-yl)-1,3,5-triazine-2,4-diyl)bis(2-methylbenzene-1,3-diol).

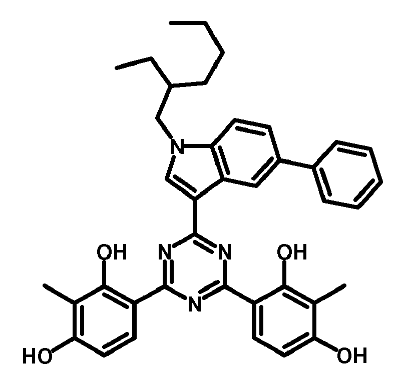

This compound was synthesized following the general procedure described above (see part 1a)) using 3-(4,6-dichloro-1,3,5-triazin-2-yl)-1-(2-ethylhexyl)-5-phenyl-1H-indole and purifying by column chromatography (41 % yield, yellow solid).

¹H-NMR (300 MHz, CDCl₃): δ 0.85 (m, 6H), 1.27 (m, 8H), 1.97 (m, 1H), 2.09 (s, 6H), 4.30 (d, J = 7.5 Hz, 2H), 6.60 (d, J = 9 Hz, 2H), 7.39 (d, J = 7.5 Hz, 1H), 7.50 (m, 2H), 7.70 (m, 2H), 7.87 (d, J = 7.5 Hz, 2H), 8.14 (d, J = 9 Hz, 2H), 8.57 (s, 1H), 8.75 (s, 1H), 10.36 (s, 2H), 13.90 (s, 2H).

### d) 6,6'-(6-(1-(2-Ethylhexyl)-5-phenyl-1H-indol-3-yl)-1,3,5-triazine-2,4-diyl)bis(3-(2-ethylhexyloxy)-2-methylphenol).

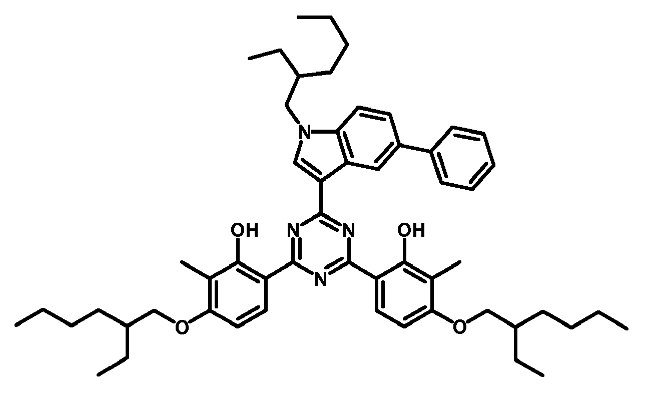

This compound was synthesized following the general procedure described above (see part 1b)) using 4,4'-(6-(1-(2-ethylhexyl)-5-phenyl-1H-indol-3-yl)-1,3,5-triazine-2,4-diyl)bis(2-methylbenzene-1,3-diol) and purifying by column chromatography (68 % yield, yellowish oil).
¹H-NMR (300 MHz, CDCl₃): δ 0.95 (m, 18H), 1.38 (m, 16H), 1.53 (m, 8H), 1.80 (m, 2H), 2.03 (m, 1H), 2.26 (s, 6H), 3.99 (d, J = 5.5 Hz, 4H), 4.12 (m, 2H), 6.59 (d, J = 9 Hz, 2H), 7.40 (m, 1H), 7.51 (m, 3H), 7.63 (m, 1H), 7.90 (d, J = 7.5 Hz, 2H), 8.15 (s, 1H), 8.42 (bs, 1H), 8.96 (bs, 2H), 13.98 (s, 2H).
UV: λₘₐₓ = 355 nm; εₘₐₓ = 51000 M⁻¹ cm⁻¹ (CH₂Cl₂).
The UV profile and λₘₐₓ show a boosted absorption of UV radiation in the UVA region, together with a remarkable absorption in the UVB region which lacks in most of UVA absorbers. Furthermore, its power of absorption is significantly greater to those of the UVA absorbers in the market. The substitution in the heterocyclic ring allows a better covering in the UVB region, without any loss in covering the UVA region (see Fig. 2B).

### Example 12.

### (12) Synthesis of 6,6'-(6-(1H-pyrazol-1-yl)-1,3,5-triazine-2,4-diyl)bis(3-(2-ethylhexyloxy)-2-methylphenol).

### a) 2,4-dichloro-6-(1H-pyrazol-1-yl)-1,3,5-triazine.

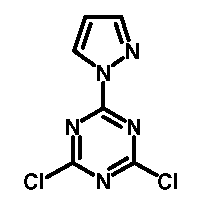

To a solution of cyanuric chloride (4 g, 21.69 mmol, 1.5 equiv.) in anhydrous THF (40 mL) at 0°C, K₂CO₃ (3.99 g, 28.92 mmol, 2 equiv.) were added. Afterwards, a solution of 1*H*-pyrazole (0.98, 14.46 mmol, 1 equiv.) in anhydrous THF (15 mL) was added dropwise. The solution is allowed to warm and then refluxed for 16 hours. The suspension formed was filtered and the filtrate evaporated. The crude residue was suspended in H₂O and extracted with CH₂Cl₂. The organic layer was dried over Na₂SO₄ anh. and evaporated. The residue was purified by column chromatography eluting petroleum ether / AcOEt 2:1, to furnish 2,4-dichloro-6-(1H-pyrazol-1-yl)-1,3,5-triazine (2.66 g, 85 % yield, white solid).

¹H-NMR (300 MHz, CDCl₃): δ 6.61 (dd, J = 3, 1.5 Hz, 1H), 7.95 (m, 1H), 8.57 (dm, J = 3 Hz, 1H).

### b) 4,4'-(6-(1H-Pyrazol-1-yl)-1,3,5-triazine-2,4-diyl)bis(2-methylbenzene-1,3-diol).

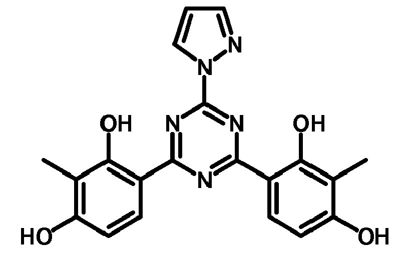

This compound was synthesized following the general procedure described above (see part 1a)) using 2,4-dichloro-6-(1H-pyrazol-1-yl)-1,3,5-triazine (46 % yield, yellow solid).

¹H-NMR (300 MHz, CDCl₃): δ 2.07 (s, 6H), 6.44 (d, J= 9 Hz, 2H), 6.64 (m, 1H), 7.91 (m, 1H), 7.98 (d, J = 9 Hz, 2H), 8.66 (d, J = 3 Hz, 1H).

### c) 6,6'-(6-(1H-Pyrazol-1-yl)-1,3,5-triazine-2,4-diyl)bis(3-(2-ethylhexyloxy)-2-methylphenol).

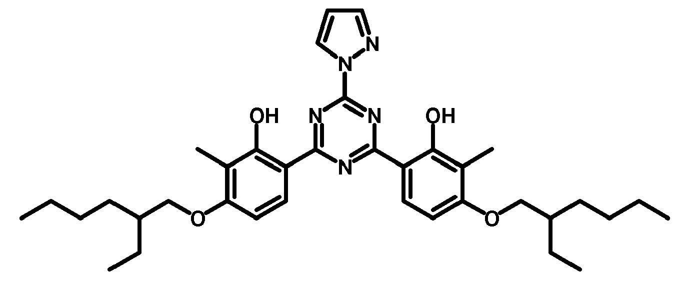

This compound was synthesized following the general procedure described above (see part 1b)) using 4,4'-(6-(1H-pyrazol-1-yl)-1,3,5-triazine-2,4-diyl)bis(2-methylbenzene-1 ,3-diol) and purifying by column chromatography (77 % yield, yellowish oil).
¹H-NMR (300 MHz, CDCl₃): δ 0.95 (m, 12H), 1.35 (m, 8H), 1.51 (m, 8H), 1.77 (m, 2H), 2.20 (s, 6H), 3.96 (m, 4H), 6.53 (d, J = 9 Hz, 1H), 6.59 (m, 2H), 7.93 (d, J = 1.5 Hz, 1H), 8.23 (bs, 2H), 8.66 (d, J = 1.5 Hz, 1H), 13.39 (s, 2H).
UV: λₘₐₓ = 340 nm; εₘₐₓ = 36000 M⁻¹ cm⁻¹ (CH₂Cl₂).
The UV profile and λₘₐₓ show a balanced absorption of UV radiation in both the UVB and UVA regions (see Fig. 2B).

### Example 13.

### (13) Synthesis of 6,6'-(6-(4-methyl-3-phenyl-1h-pyrazol-1-yl)-1,3,5-triazine-2,4-diyl)bis(3-(2-ethylhexyloxy)-2-methylphenol).

### a) 2,4-dichloro-6-(4-methyl-3-phenyl-1h-pyrazol-1-yl)-1,3,5-triazine.

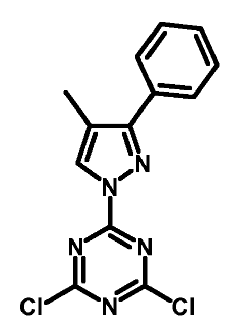

This compound was synthesized following the general procedure described above (see part (12)a)) using 4-methyl-3-phenyl-1H-pyrazole (99 % yield, yellow solid).

¹H-NMR (300 MHz, CDCl₃): δ 2.54 (s, 3H), 7.39 (m, 1H), 7.46 (m, 5H).

### b) 4,4'-(6-(4-Methyl-3-phenyl-1H-pyrazol-1-yl)-1,3,5-triazine-2,4-diyl)bis(2-methylbenzene-1,3-diol).

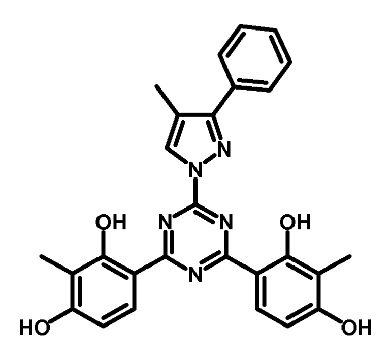

This compound was synthesized following the general procedure described above (see part 1a)) using 2,4-dichloro-6-(4-methyl-3-phenyl-1H-pyrazol-1-yl)-1,3,5-triazine (89 % yield, yellow solid).

¹H-NMR (300 MHz, CDCl₃): δ 2.05 (s, 6H), 2.55 (s, 3H), 6.61 (d, J= 9 Hz, 2H), 7.38 (m, 2H), 7.48 (m, 3H), 7.67 (d, J = 6.5 Hz, 2H), 8.11 (m, 1H), 10.50 (s, 2H), 13.52 (s, 2H).

### c) 6,6'-(6-(4-Methyl-3-phenyl-1H-pyrazol-1-yl)-1,3,5-triazine-2,4-diyl)bis(3-(2-ethylhexyloxy)-2-methylphenol).

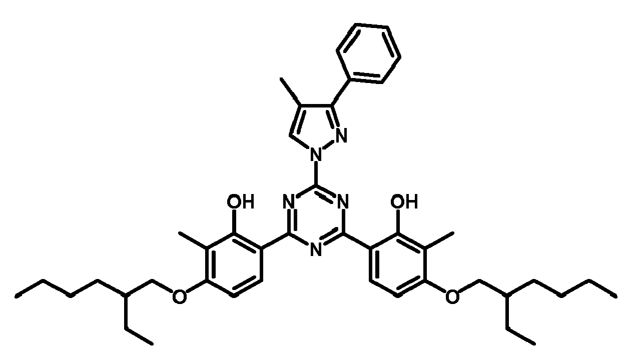

This compound was synthesized following the general procedure described above (see part 1b)) using 4,4'-(6-(4-methyl-3-phenyl-1H-pyrazol-1-yl)-1,3,5-triazine-2,4-diyl)bis(2-methylbenzene-1,3-diol) and purifying by column chromatography (23 % yield, yellowish oil).
¹H-NMR (300 MHz, CDCl₃): δ 0.95 (m, 12H), 1.35 (m, 8H), 1.51 (m, 8H), 1.78 (m, 2H), 2.21 (s, 6H), 2.55 (s, 3H), 3.97 (m, 4H), 6.58 (d, J = 9 Hz, 2H), 7.49 (m, 5H), 8.25 (bs, 2H), 8.65 (s, 1H), 13.41 (s, 2H).
UV: λₘₐₓ = 326 nm; εₘₐₓ = 51000 M⁻¹ cm⁻¹ (CH₂Cl₂).
The UV profile and λₘₐₓ show absorption of UV radiation in between the UVB and UVA regions, boosted by the substitution in the heterocyclic ring. As a result, this UV filter acts in the inter-phase of most UVB and UVA filters (see Fig. 2B).

### Example 14.

### (14) Synthesis of 6,6'-(6-(4-(4-methoxyphenyl)-1H-pyrazol-1-yl)-1,3,5-triazine-2,4-diyl)bis(3-(2-ethylhexyloxy)-2-methylphenol).

### a) 2,4-dichloro-6-(4-(4-methoxyphenyl)-1H-pyrazol-1-yl)-1,3,5-triazine.

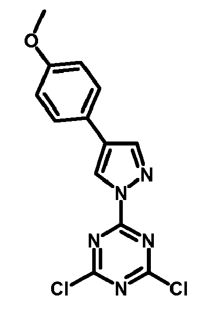

This compound was synthesized following the general procedure described above (see part (12)a)) using 4-(4-methoxyphenyl)-1H-pyrazole (70 % yield, yellow solid).

¹H-NMR (300 MHz, CDCl₃): δ 3.85 (s, 3H), 6.97 (dm, J= 9 Hz, 2H), 7.53 (dm, J= 9 Hz, 2H), 8.20 (d, J= 1 Hz, 1H), 8.67 (d, J= 1 Hz, 1H).

### b) 4,4'-(6-(4-(4-Methoxyphenyl)-1H-pyrazol-1-yl)-1,3,5-triazine-2,4-diyl)bis(2-methylbenzene-1,3-diol).

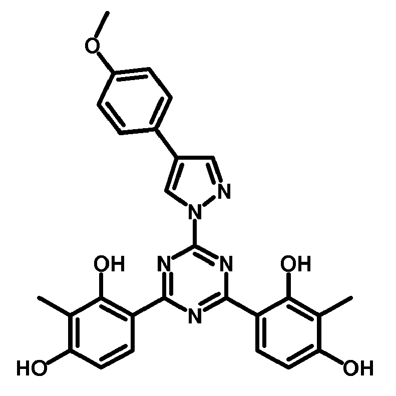

This compound was synthesized following the general procedure described above (see part 1a)) 2,4-dichloro-6-(4-(4-methoxyphenyl)-1H-pyrazol-1-yl)-1,3,5-triazine (91 % yield, yellow solid).

¹H-NMR (300 MHz, CDCl₃): δ 2.06 (s, 6H), 3.80 (s, 3H), 6.64 (d, J = 9 Hz, 2H), 7.01 (d, J = 9 Hz, 2H), 7.83 (d, J = 9 Hz, 2H), 8.16 (d, J = 9 Hz, 2H), 8.53 (s, 1H), 9.32 (s, 1H), 10.52 (s, 2H), 13.56 (s, 2H).

### c) 6,6'-(6-(4-(4-Methoxyphenyl)-1H-pyrazol-1-yl)-1,3,5-triazine-2,4-diyl)bis(3-(2-ethylhexyloxy)-2-methylphenol).

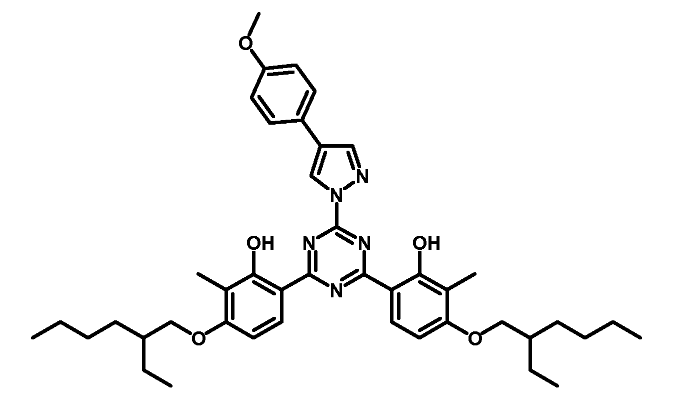

This compound was synthesized following the general procedure described above (see part 1b)) using 4,4'-(6-(4-(4-methoxyphenyl)-1H-pyrazol-1-yl)-1,3,5-triazine-2,4-diyl)bis(2-methylbenzene-1,3-diol) and purifying by column chromatography (15 % yield, yellowish oil).

¹H-NMR (300 MHz, CDCl₃): δ 0.97 (m, 12H), 1.37 (m, 8H), 1.51 (m, 8H), 1.80 (m, 2H), 2.23 (s, 6H), 3.88 (s, 3H), 3.99 (d, J = 5.5 Hz, 4H), 6.61 (d, J = 9 Hz, 2H), 6.99 (d, J = 9 Hz, 2H), 7.58 (d, J = 9 Hz, 2H), 8.17 (s, 1H), 8.29 (bs, 2H), 8.77 (s, 1H), 13.46 (s, 2H). UV: λₘₐₓ = 343 nm; εₘₐₓ = 54000 M⁻¹ cm⁻¹ (CH₂Cl₂). The UV profile and λₘₐₓ show a boosted absorption of UV radiation in the UVA region, together with a remarkable absorption in the UVB region which lacks in most of UVA absorbers. Furthermore, its power of absorption is significantly greater to those of the UVA absorbers in the market. The substitution in the heterocyclic ring boosts both the covering in the UVA region and its power of absorption (see Fig. 2B).

### Example 15.

### Photostability tests

### a) Procedure of Photo-stability assays.

A 3 % w/v solution in Miglyol 812 of the compound to analyze was made (standard sample). 4 samples of this solution were made from aliquots and were introduced in glass weighing bottles. Two of them were protected with quartz covers and irradiated with UV light, for the period of time scheduled, at a controlled temperature of 35°C and a power of irradiation of 765 W*h/m² (test samples). The 2 remaining weighing bottles were protected from UV light with aluminium foil and maintained close to irradiated samples into Suntest chamber (control samples).

After the irradiation period, all test and control samples were analyzed. The amount of UV filter/s of processed test, control and standard samples were quantified by HPLC.

### b) Photo-stability assays of single compounds.

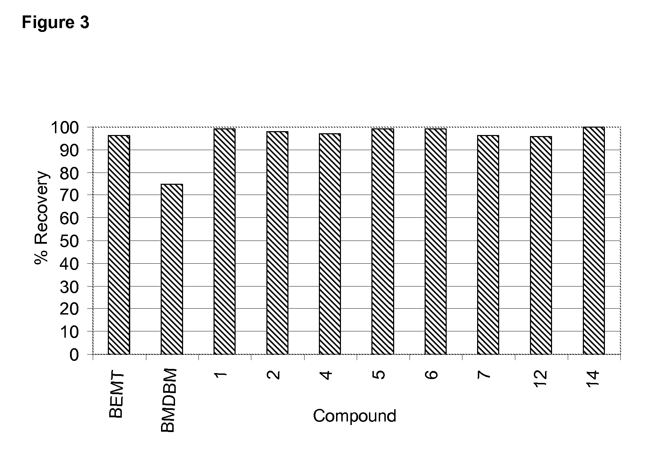

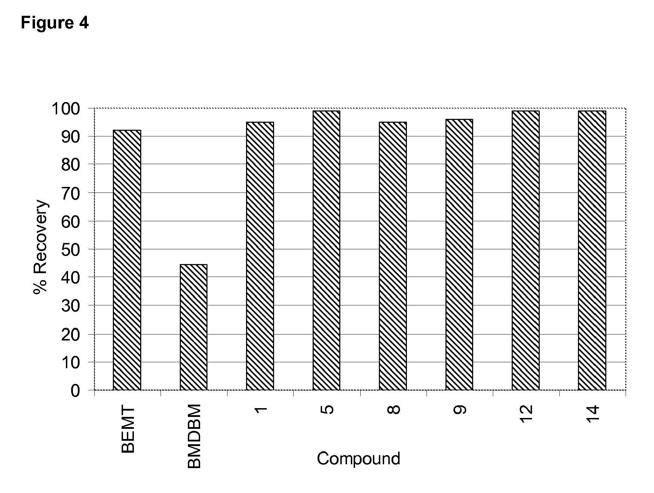

Several compounds from the Examples given above were irradiated for 2 and 4 hours showing excellent photo-stability. The percentages of recovery were superior to 90 %, threshold that is the univocal sign of the great photo-stability of the compounds synthesized. Furthermore, the percentages of recovery of these compounds were even better of those of commercial compounds like *bis*-ethylhexyloxyphenol methoxyphenyl triazine (BEMT) and *tert*-butyl methoxydibenzoyl methane (BMDBM) (cf. Figures 3 and 4).

### c) Photo-stability assays in combination with ethylhexyl methoxycinnamate (EHMC).

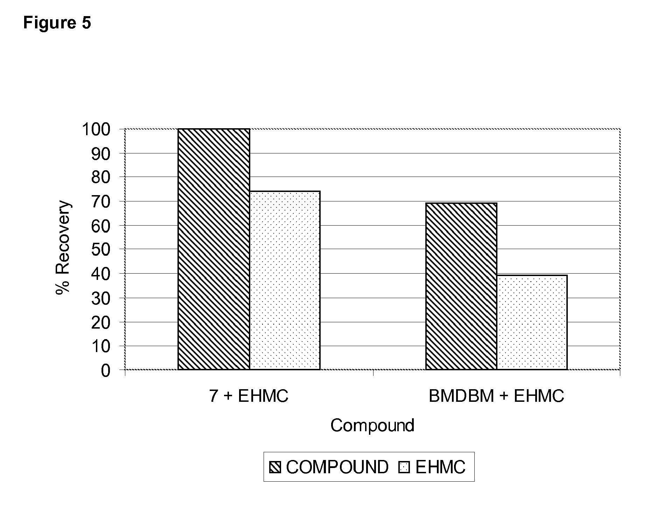

Unlike BMDBM, compounds with a boosted UVA absorption profile (e.g. compound from example 7) irradiated in combination with ethylhexyl methoxycinnamate for 2 hours showed excellent photo-stability, even protecting ethylhexyl methoxycinnamate in front of photo-degradation. The Figure 5 shows the results.

### d) Photo-stability assays in combination with tert-butyl methoxydibenzoyl methane.

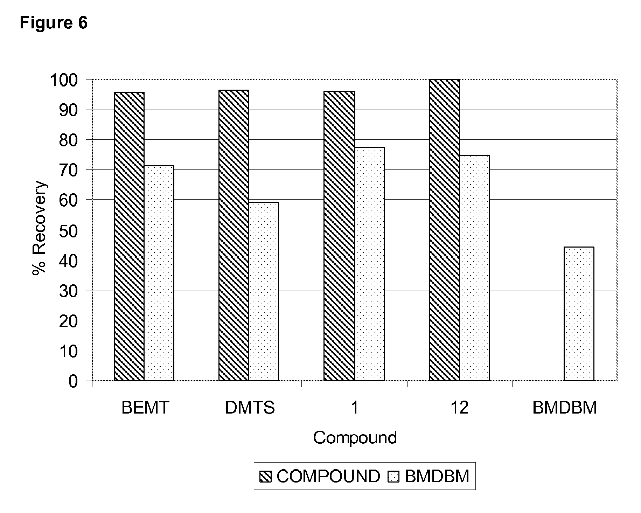

Compounds of Example 1 and 12 were irradiated in combination with *tert-*butyl methoxydibenzoyl methane (BMDBM) for 4 hours showing excellent photo-stability. The Figure 6 shows not only the recovery of the compound analyzed but also the recovery of *tert*-butyl methoxydibenzoyl methane itself. The percentage of recoveries of the compound analyzed were superior to 90 %, showing the great photo-stability of the compounds even when they are irradiated in combination with a UV filter with low photo-stability. Moreover, our compounds are capable of making *tert*-butyl methoxydibenzoyl methane more photo-stable when combined with them, within a synergistic protection process. In addition, our compounds showed superior photo-stabilization of *tert*-butyl methoxydibenzoyl methane than the brought by commercial compounds like *bis*-ethylhexyloxyphenol methoxyphenyl triazine (BEMT) and drometrizole trisiloxane (DMTS) (Figure 6).

## Claims

1. A compound of formula (I), a stereoisomer thereof, a pharmaceutically, cosmetically or dermatologically acceptable salt thereof or a pharmaceutically, cosmetically or dermatologically acceptable solvate thereof: 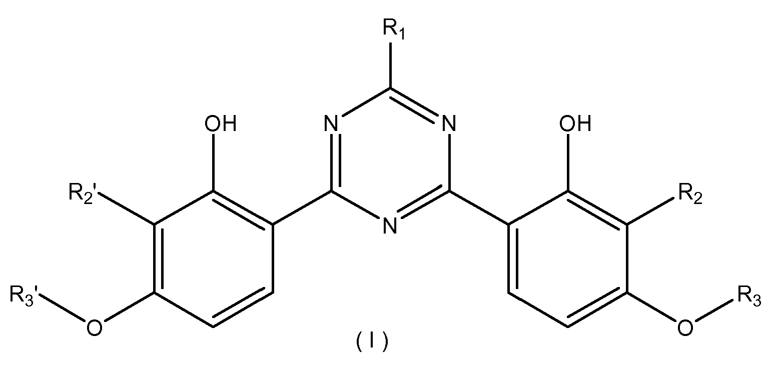 wherein:
R₁ is a ring system of formula (II): 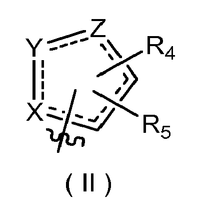
wherein:
X, Y and Z are each one independently selected from C and N, being at least one of them different from C;
the dotted line represents the presence or absence of a double bond;
the wavy line shows the attach point of the moiety;
R₄ and R₅ are independently selected from H, halogen, nitro, cyano, linear or branched (C₁-C₁₈)alkyl, linear or branched (C₂-C₁₈)alkenyl, -OR₆, -COR₆, -COOR₆, -OC(O)R₆, -C(O)NR₇R₈, -R₉NR₇R₈, -R₉PO(OR₁₀)₂ -S-R₁₁, -SO-R₁₁, -SO₂-R₁₁, -SO₃M, -NHSO₂R₁₁, -SO₂-NR₇R₈, -NR₇R₈, and an optionally substituted aromatic or heteroaromatic ring having 5-6 members; being the substituent selected from the group consisting of halogen, nitro, cyano, linear or branched (C₁-C₈)alkyl, linear or branched (C₂-C₈)alkenyl, -OR₆, -COR₆, -COOR₆, -OC(O)R₆, -C(O)NR₇R₈, -R₉NR₇R₈, -R₉PO(OR₁₀)₂ -S-R₁₁, -SO-R₁₁, -SO₂-R₁₁, -SO₃M, -NHSO₂R₁₁, -SO₂-NR₇R₈, and -NR₇R₈;
or R₄ and R₅ taken together with the atoms to which they are attached form one of the known ring of 6 members, saturated, partially unsaturated or aromatic, wherein each member is independently selected from C, and N; thereby the ring system of formula (II) is a bicyclic system;
the bicyclic system of formula (II) being optionally substituted by at least one radical selected from the group consisting of halogen, nitro, cyano, linear or branched (C₁-C₁₈)alkyl, linear or branched (C₂-C₁₈)alkenyl, -OR₆, -COR₆, -COOR₆, -OC(O)R₆, -C(O)NR₇R₈, -R₉NR₇R₈, -R₉PO(OR₁₀)₂ -S-R₁₁, -SO-R₁₁, -SO₂-R₁₁, -SO₃M, -NHSO₂-R₁₁, -SO₂-NR₇R₈, -NR₇R₈, and an optionally substituted aromatic or heteroaromatic ring having 5-6 members; being the substituent selected from the group consisting of halogen, nitro, cyano, linear or branched (C₁-C₈)alkyl, linear or branched (C₂-C₈)alkenyl, -OR₆, -COR₆, -COOR₆, -OC(O)R₆, -C(O)NR₇R₈, -R₉NR₇R₈, -R₉PO(OR₁₀)₂ -S-R₁₁, -SO-R₁₁, -SO₂-R₁₁, -SO₃M, -NHSO₂R₁₁, -SO₂-NR₇R₈, and -NR₇R₈;
R₆, R₇, R₈, R₁₀ and R₁₁ are radicals, same or different, independently selected from the group consisting of hydrogen, and linear or branched (C₁-C₆)alkyl;
R₉ is a biradical selected from the group consisting of linear or branched (C₁-C₆)alkyl;
R₂ and R₂' are radicals, same or different, independently selected from the group consisting of linear or branched (C₁-C₄)alkyl, and -O-(C₁-C₄)alkyl ;
R₃ and R₃' are radicals, same or different, independently selected from the group consisting of hydrogen, (C₁-C₁₈)acyl radical, linear or branched (C₁-C₁₈)alkyl, and linear or branched (C₂-C₁₈)alkenyl; wherein the radical is optionally substituted with at least one radical selected from the group consisting of OH, -SO₃M, -N(R₁₂)₃⁺ X⁻ group and a group of general formula (III) 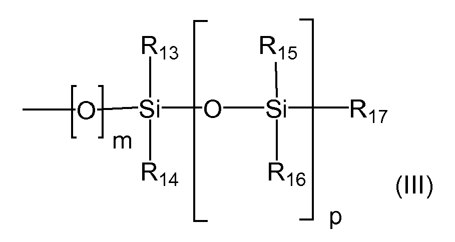
in which
m= 0 or 1;
p= 0, 1, 2, 3 or 4;
R₁₃, R₁₄, R₁₅, R₁₆ and R₁₇ are independently selected from the group consisting of (C₁-C₆)alkyl radical; an (C₁-C₆)alkoxy radical and an -OSi(R₁₈)₃ radical;
R₁₈ represents an (C₁-C₆)alkyl radical or an (C₁-C₆)alkoxy radical;
R₁₂ represents an (C₁-C₆)alkyl radical; and
M is H, Na, K or triethanolamine;
X⁻ represents an anion of a dermatologically, cosmetically, and/or pharmaceutically acceptable acid.

2. The compound according to claim 1, wherein R₁ is selected from the group consisting of 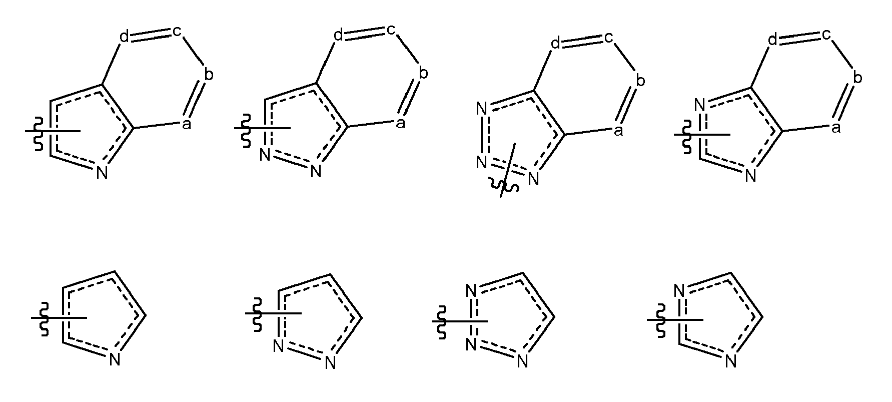 wherein a, b, c, and d are independently selected from C, and N;
the wavy line shows the attach point of the moiety;
wherein R₁ is optionally substituted by at least one radical selected from the group consisting of halogen, nitro, cyano, linear or branched (C₁-C₈)alkyl, linear or branched (C₂-C₈)alkenyl, -OR₆, -COR₆, -COOR₆, -OC(O)R₆, -C(O)NR₇R₈, -R₉NR₇R₈, -R₉PO(OR₁₀)₂, -S-R₁₁, -SO-R₁₁, -SO₂-R₁₁, -SO₃M, -NHSO₂R₁₁, -SO₂-NR₇R₈, -NR₇R₈, and an optionally substituted aromatic or heteroaromatic ring having 5-6 members; being the substituent selected from the group consisting of halogen, nitro, cyano, linear or branched (C₁-C₈)alkyl, linear or branched (C₂-C₈)alkenyl, -OR₆, -COR₆, -COOR₆, -OC(O)R₆, -C(O)NR₇R₈, -R₉NR₇R₈, -R₉PO(OR₁₀)₂-S-R₁₁, -SO-R₁₁, -SO₂-R₁₁, -SO₃M, -NHSO₂R₁₁, -SO₂-NR₇R₈, and -NR₇R₈.

3. The compound according to claim 2, wherein R₁ is selected from the group consisting of

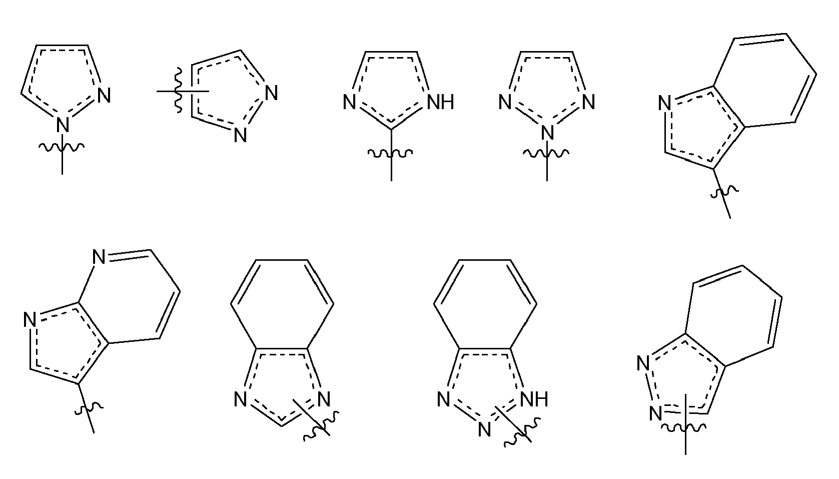

4. The compound according to claim 3, wherein R₁ is selected from the group consisting of

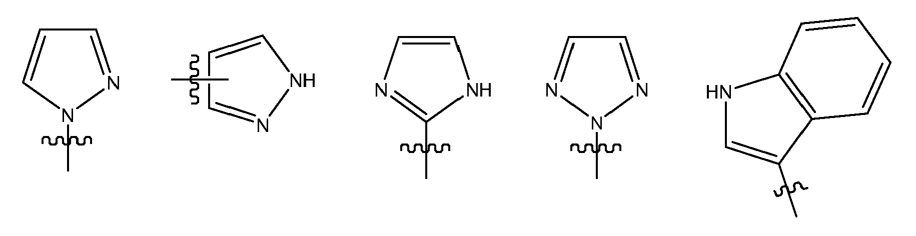

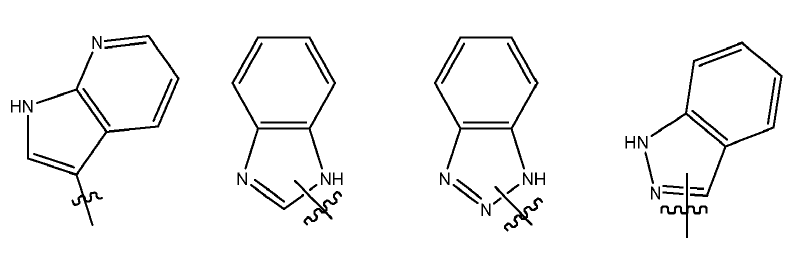

5. The compound according to any of claims 1 to 4, wherein R₁ is substituted by at least one radical selected from the group consisting of halogen, linear or branched (C₁-C₈)alkyl, -OR₆, -NR₇R₈, and an optionally substituted aromatic or heteroaromatic ring having 5-6 members; being the substituent selected from the group consisting of halogen, cyano, linear or branched (C₁-C₈)alkyl, -OR₆, -COR₆, -NR₇R₈, and -SO₃M.

6. The compound according to claim 5 wherein R₁ is substituted by at least one radical selected from the group consisting of methyl, ethyl, propyl, iso-propyl, OH, methoxy, ethoxy, 2-ethylhexyl, 2-ethylhexyloxy, phenyl optionally substituted by at least one radical selected from OH, methyl, ethyl, methoxy, ethoxy, dimethylamino, -SO₃M, and -NH₂.

7. The compound according to any of claims 1 to 6, wherein R₂ and R₂' are radicals, same or different, independently selected from the group consisting of methyl, ethyl, propyl, *iso*-propyl, methoxy, and ethoxy.

8. The compound according to any of claims 1 to 7, wherein R₃ and R₃' are radicals, same or different, independently selected from the group consisting of methyl, ethyl, propyl, *iso*-propyl, butyl, *tert*-butyl, *iso*-amyl, hexyl, 2-ethylhexyl, 2-ethyloctyl, 2-butyloctyl, 2-hexyldecyl, -CO-methyl, -CO-ethyl, -CO-propyl, -CO-*iso*-propyl, -CO-butyl, -CO-*tert*-butyl, -CO-hexyl, -CO-octyl; wherein the radical is optionally substituted with at least one radical selected from the group consisting of OH, -SO₃M and -N(Me)₃⁺ X⁻ group or else a group of general formula (III)

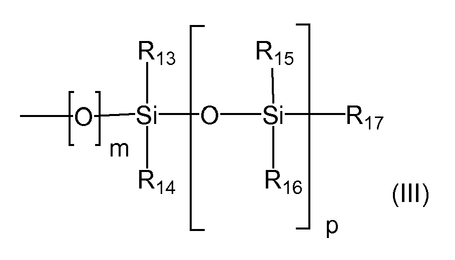

9. The compound according to claim 8, wherein R₃ and R₃' are radicals, same or different, independently selected from the group consisting of methyl, ethyl, propyl, *iso*-propyl, butyl, *tert*-butyl, *iso*-amyl, hexyl, 2-ethylhexyl, 2-butyloctyl, 2-hexyldecyl.

10. The compound according to any of claims 1 to 9, which is selected from the group consisting of:
(1) 2-(1-methyl-pyrazol-5-yl)-4,6-bis[4-(2-ethylhexyloxy)-3-methyl-2-hydroxy-phenyl]-1,3,5-triazine.
(2) 2-(1-methyl-pyrazol-5-yl)-4,6-bis[4-(2-butyloctyloxy)-3-methyl-2-hydroxy-phenyl]-1,3,5-triazine.
(3) 6,6'-(6-(1-methyl-1H-indol-3-yl)-1,3,5-triazine-2,4-diyl)bis(3-(2-ethylhexyloxy)-2-methylphenol).
(4) 6,6'-(6-(1-methyl-1H-indol-3-yl)-1,3,5-triazine-2,4-diyl)bis(3-(2-butyloctyloxy)-2-methylphenol).
(5) 6,6'-(6-(1-(2-ethylhexyl)-1H-indol-3-yl)-1,3,5-triazine-2,4-diyl)bis(3-(2-ethylhexyloxy)-2-methylphenol).
(6) 6,6'-(6-(1-(2-ethylhexyl)-1H-indol-3-yl)-1,3,5-triazine-2,4-diyl)bis(3-(2-butyloctyloxy)-2-methylphenol).
(7) 6,6'-(6-(1-(2-ethylhexyl)-5-methoxy-1H-indol-3-yl)-1,3,5-triazine-2,4-diyl)bis(3-(2-ethylhexyloxy)-2-methylphenol).
(8) 6,6'-(6-(1-(2-ethylhexyl)-2-methyl-1H-indol-3-yl)-1,3,5-triazine-2,4-diyl)bis(3-(2-ethylhexyloxy)-2-methylphenol).
(9) 6,6'-(6-(1-(2-ethylhexyl)-5-methoxy-2-methyl-1H-indol-3-yl)-1,3,5-triazine-2,4-diyl)bis(3-(2-ethylhexyloxy)-2-methylphenol).
(10) 6,6'-(6-(1-(2-ethylhexyl)-5-(4-methoxyphenyl)-1H-indol-3-yl)-1,3,5-triazine-2,4-diyl)bis(3-(2-ethylhexyloxy)-2-methylphenol).
(11) 6,6'-(6-(1-(2-ethylhexyl)-5-phenyl-1H-indol-3-yl)-1,3,5-triazine-2,4-diyl)bis(3-(2-ethylhexyloxy)-2-methylphenol).
(12) 6,6'-(6-(1H-pyrazol-1-yl)-1,3,5-triazine-2,4-diyl)bis(3-(2-ethylhexyloxy)-2-methylphenol).
(13) 6,6'-(6-(4-methyl-3-phenyl-1H-pyrazol-1-yl)-1,3,5-triazine-2,4-diyl)bis(3-(2-ethylhexyloxy)-2-methylphenol).
(14) 6,6'-(6-(4-(4-methoxyphenyl)-1H-pyrazol-1-yl)-1,3,5-triazine-2,4-diyl)bis(3-(2-ethylhexyloxy)-2-methylphenol).

11. Compound according to any of claims 1 to 10 for use as a UV radiation-absorbing agent.

12. Dermatological, cosmetic, pharmaceutical or veterinary formulation comprising one or more compounds according to claim 1 and at least one dermatologically, cosmetically, and/or pharmaceutically acceptable carrier or excipients.

13. Formulation according to claim 12, which further includes at least one organic, micronized organic or inorganic filter against solar radiation, or one active substance.

14. Use of a compound according to any of claims 1 to 10, in a cosmetic, dermatological, veterinary or pharmaceutical composition as a UV radiation filtering agent; for manufacturing a formulation to protect the skin, lips and/or related tissues of a mammal against ultraviolet radiation; or for manufacturing a formulation for preventive use, as a coadjuvant in the treatment of pathologies caused by ultraviolet radiation on the skin, lips and/or related tissues of a mammal.

15. Use of a derivative according to any of claims 1 to 10, as a photostabiliser of polymers, or as an ultraviolet radiation-filtering agent in textile fibres.
